# EUROPEAN PATENT APPLICATION

(11) **EP 2 913 671 A1**
(43) Date of publication of application: **02.09.2015**
(21) Application number: 14157002.8
(22) Date of filing: 27.02.2014
(51) Int. Cl.: G01N 33/543, A61L 33/00, A61M 1/36, A61L 2/00

(54) **Novel coated carriers for the specific binding of target molecules**

(71) Applicant: LEUKOCARE AG, 82152 Martinsried / München (DE)
(72) Inventor: Kirchner, Roland, 83533 Edling (DE); Altrichter, Jens, 18196 Kavelstorf (DE); Scholz, Martin, 80339 München (DE)
(74) Representative: Vossius & Partner Patentanwälte Rechtsanwälte mbB

(57) **Abstract**

The present invention relates to a method of producing a solid carrier, to which at least one affinity-reagent is coupled that binds specifically to a target molecule, comprising the steps of (i) coupling at least one affinity-reagent to a solid carrier, (ii) overlaying the affinity-reagent-coupled carrier obtained in (i) by incubation with a composition comprising heparin and/or a functional derivative thereof, and (iii) overlaying the affinity-reagent-coupled carrier by incubation with a composition comprising stabilising excipients, wherein steps (ii) and (iii) are carried out subsequently or simultaneously and subsequently to step (i). The invention further relates to a solid carrier comprising (i) at least one affinity-reagent, wherein the at least one affinity-reagent is coupled to the solid carrier, (ii) an overlay comprising heparin and/or a functional derivative thereof, and (ii) an overlay comprising stabilising excipients. In addition, the invention relates to the use of the carrier of the invention or of the carrier produced by the method of the invention to specifically bind and enrich a target molecule or a target cell which carries a biological target molecule on its surface from a liquid sample. Further, the invention relates to the use of the carrier of the invention or of the carrier produced by the method of the invention to specifically induce a biological response in a target cell. Furthermore, the invention relates to an *ex-vivo* or *in-vitro* method for specifically binding and enriching a target cell out of a liquid sample comprising contacting the liquid sample with the carrier of the invention or the carrier produced by the method of the invention, wherein the contacting is effected by inducing the liquid sample to flow over the surface of the carrier to which the at least one affinity-reagent is coupled, and wherein the target cell carries a biological target molecule on its surface to which the at least one affinity-reagent specifically binds. Moreover, the invention relates to the carrier of the invention or the carrier produced by the methods of the invention for use as an implant or as an extracorporeal device.

## Description

The present invention relates to a method of producing a solid carrier, to which at least one affinity-reagent is coupled that binds specifically to a target molecule, comprising the steps of (i) coupling at least one affinity-reagent to a solid carrier, (ii) overlaying the affinity-reagent-coupled carrier obtained in (i) by incubation with a composition comprising heparin and/or a functional derivative thereof, and (iii) overlaying the affinity-reagent-coupled carrier by incubation with a composition comprising stabilising excipients, wherein steps (ii) and (iii) are carried out subsequently or simultaneously and subsequently to step (i). The invention further relates to a solid carrier, comprising (i) at least one affinity-reagent, wherein the at least one affinity-reagent is coupled to the solid carrier, (ii) an overlay comprising heparin and/or a functional derivative thereof, and (ii) an overlay comprising stabilising excipients. In addition, the invention relates to the use of the carrier of the invention or of the carrier produced by the method of the invention to specifically bind and enrich a target molecule or a target cell which carries a biological target molecule on its surface from a liquid sample. Further, the invention relates to the use of the carrier of the invention or of the carrier produced by the method of the invention to specifically induce a biological response in a target cell. Furthermore, the invention relates to an *ex-vivo* or *in-vitro* method for specifically binding and enriching a target cell out of a liquid sample comprising contacting the liquid sample with the carrier of the invention or the carrier produced by the method of the invention, wherein the contacting is effected by inducing the liquid sample to flow over the surface of the carrier to which the at least one affinity-reagent is coupled, and wherein the target cell carries a biological target molecule on its surface to which the at least one affinity-reagent specifically binds. Moreover, the invention relates to the carrier of the invention or the carrier produced by the methods of the invention for use as an implant or as an extracorporeal device.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The formation of metastases is the major cause of death from cancer. Tumour cells spread to secondary sites late in tumourigenesis. Tumour cell dissemination profiles seem to largely differ in different cancer diseases. For example, in about 30% of breast cancer patients, micro-metastases could be detected in the bone marrow at the time of diagnosis, regardless of the disease stage (Braun, Vogl et al. 2005) and prostate cancer cells disseminate early from the primary tumour (Morgan, Lange et al. 2009). Karyotypic abnormalities of micro-metastasis in bone marrow of breast cancer patients as well as in animal models indicate that tumour cell dissemination occurs in the pre-invasive stage of disease progression (Husemann, Geigl et al. 2008). Overall, it is highly evident that it is not sufficient to characterise cells from the primary tumour in order to determine the probability of disease recurrence and to determine the appropriate therapeutic strategy. The analysis of tumour cells that were disseminated into the bone marrow should be included as a diagnostic tool (Pantel and Brakenhoff 2004).

In the light of individualised medicine, more specific and optimised therapeutic strategies are urgently needed. Drugs that specifically target cells, in particular cancer cells, are supposed to be more effective with less adverse side effects and less drug resistance. In order to develop highly specific chemotherapy strategies, the tumour cells ought to be well characterised. In the past, biopsies from solid tumours were used for diagnosis. However, the relevance of these biopsies for the diagnosis of tumour progression is still unclear because metastasis-forming tumour cells might not be found. There is increasing evidence that the amount and character of circulating tumour cells (CTCs) in the peripheral blood circulation of cancer patients may be more important for diagnosis of disease progression. CTCs are cells that detach from a primary tumour or metastatic site, circulate in the peripheral blood and may form metastasis. Consequently, CTCs in a blood sample may differ from tumour cells in the solid tumour and thus may provide important additional information for patient specific therapy. Currently, the difference between tumour cells obtained from solid tumours by biopsies and circulating tumour cells is poorly defined. Phenotypic and molecular analyses of CTCs may be useful for diagnostic and therapeutic purposes and may provide a better risk assessment and more specific therapeutic decisions in the treatment of cancer patients. Phenotyping and genotyping CTCs may be helpful for the scientific understanding of mechanisms involved in the formation of metastases, including immune escape mechanisms, transendothelial migration, tissue targeting etc. and the development of future therapy strategies. A comprehensive understanding of the genome and transcriptome in CTCs might lead to the development of novel predictive markers and the identification of additional therapeutic targets. In addition, the analysis of CTCs from peripheral blood as a liquid biopsy would avoid multiple needle biopsies that are highly invasive (Muller and Pantel 2004).

To date, CTCs are used for diagnosis by analysing a small blood volume that has been obtained by drawing blood with a standard syringe. However, CTCs are rare events (average 1 CTC per 10⁸ leukocytes in 10 ml peripheral blood). In some patients, the probability to detect CTCs may therefore be extremely low and may give false negative results even with the most commonly employed system, the CellSearch system (Amadori, Rossi et al. 2009). Altogether, in order to enable the development of valuable diagnostics, cell characterisation, and better therapeutic strategies, there is a need to increase the amount of captured cells.

At present, the CellSearch system is the only validated assay for CTC detection that has been cleared by the U.S. Food and Drug Administration. Prospective multicenter studies in metastatic breast, prostate, and colon cancer conducted with this system demonstrated that the presence of CTCs was associated with poor survival and failure to eliminate these CTCs after the first cycles of therapy strongly suggests futile therapy. However, the number of CTCs collected from patients using this system is too low for further and more differentiated diagnosis. For example, CTC genotyping and phenotyping may provide important information on the success of the treatment strategy. The feasibility to detect treatment targets in CTCs has been already demonstrated (Meng, Tripathy et al. 2004;de Bono, Attard et al. 2007; Rossi, Basso et al. 2010; Wang, Pfister et al. 2010). The currently used CellSearch system allows the analysis of CTCs within 7.5 ml of blood based on the expression of both EpCAM and Cytokeratin 8, 18 or 19. By using the CellSearch system, it was shown that 57% of patients with metastatic prostate (Danila, Heller et al. 2007; de Bono, Scher et al. 2008), 50% with metastatic breast (Cristofanilli, Budd et al. 2004), 18% with metastatic colorectal (Cohen, Punt et al. 2008; Sastre, Maestro et al. 2008) and 15% with metastatic lung cancer (Hofman, Ilie et al. 2011; Krebs, Sloane et al. 2011) have 5 or more CTCs in 7.5 mL of blood before initiation of a new line of therapy. In the majority of these patients, CTCs are thus not available for therapeutic profiling. By extrapolation of the CTC frequency distribution in 7.5 ml of blood from patients with metastatic breast, colon and prostate cancer it was shown that probably all these patients had CTCs in the peripheral blood. However, the sample volume was too small to detect CTCs in all patients. In addition, there is evidence that patient survival is a function of CTC frequency in peripheral blood (Cohen SJ et al. (2009); Ann Oncol.;20(7):1223-9) Therefore, not only the qualitative detection but also quantification of CTCs is of important prognostic value. In conclusion, CTCs from a significantly larger blood volume have to be obtained in order to further exclude false negative results, to be able to obtain a reliable quantification and to be able to further characterise the CTCs. Moreover, for the further characterisation of CTCs it is required that the purity of CTCs obtained from the sample is high enough.

One potential approach in obtaining a larger number of cells and/or to detect rare CTCs in patient blood for diagnostic and therapeutic reasons is to conduct a therapeutic apheresis (TA). Therefore, a TA column that specifically enriches CTCs from a large volume of peripheral blood in cancer patients by specifically binding CTCs out of the blood circulation would be a desirable solution. Importantly, the binding efficacy and the specificity of such a system ought to be sufficiently high to collect a relevant number of CTCs. Presently, a major hurdle to obtain a large number of CTCs out of body fluids, such as blood, is the specificity of the binding matrix. Unspecific binding of leukocytes, erythrocytes, and other circulating cells or molecules within the blood circulation can limit the diagnostic and therapeutic value of such a system, as the further characterisation of bound CTCs is impaired due to the lack of purity after elution out of the column. Further, a high amount of unspecific binding may lead to an undesired reduction or depletion of other cell types, which are beneficial or even required for physiological functions, from the peripheral blood of the patient. Accordingly, the unspecific binding of beneficial cells during therapeutic apheresis is undesired and potentially harmful to the patient. This also limits the use of apheresis systems in removing CTCs from the peripheral blood of a patient as a therapeutic measure.

In addition, a major concern in the manufacturing of therapeutic, prophylactic and diagnostic products, such as for example apheresis columns, is to assure that no microbial contaminants can be transmitted to the recipient or user of such products. However, these pharmaceutical products are generally sourced from biological materials which may contain pathogens like e.g. viruses or their manufacturing process makes use of reagents or culture media that are potentially contaminated with pathogens.

One solution to produce non-contaminated pharmaceuticals or medical devices is their aseptic production. However, a major drawback of this approach is that the whole production process has to be performed under aseptic conditions in a clean room. This is time consuming and cost intensive. It is therefore preferable to introduce steps into the manufacturing process that remove or inactivate those potential bacterial or viral contaminants, such as for example a sterilisation step.

The chemical, physical or physiological properties of bio-functional molecules are often significantly altered by variations in the compound's surrounding environment, as, for example, occurring during sterilisation, storage or transport. For example, changes in pH, ionic strength, or temperature as well as the exposure to irradiation or oxidizing conditions can result in reversible or irreversible changes in the character of compounds, thereby leading to a loss of activity of active compounds, especially biomolecules, such as e.g. affinity-reagents. Thus, it is desirable that such irreversible changes to these active compounds are avoided, e.g. by stabilising the active compounds.
Accordingly, although TA offers a possibility of enriching CTCs, this approach is currently hampered by the high background of blood cells and low specificity and/or stability of biomolecules employed as affinity-reagents in apheresis columns. Thus, despife considerable efforts, there is still a need for suitable and sterile systems for obtaining CTCs of high purity and in a sufficient amount.

This need is addressed by the provision of the embodiments characterised in the claims.

Accordingly, the present invention relates to a method of producing a solid carrier, to which at least one affinity-reagent is coupled that binds specifically to a target molecule, comprising the steps of (i) coupling at least one affinity-reagent to a solid carrier, (ii) overlaying the affinity-reagent-coupled carrier obtained in (i) by incubation with a composition comprising heparin and/or a functional derivative thereof, and (iii) overlaying the affinity-reagent-coupled carrier by incubation with a composition comprising stabilising excipients, wherein steps (ii) and (iii) are carried out subsequently or simultaneously and subsequently to step (i).

The terms employed herein are well known in the art and are used in accordance with the definitions provided in the art, i.e. the definitions provided in this specification are solely intended to reflect the understanding of these terms in the art. Where nevertheless a discrepancy arises, the definitions provided herein supersede.

The term "carrier", also referred to herein as "matrix", as used herein refers to a material that is used as a solid support for the affinity-reagent(s). In accordance with the present invention the term "solid carrier" refers to a carrier which is in a physical state that resists changes in its molecular structure and which is insoluble in aqueous solutions at temperatures of up to 37°C. Preferably, the solid carrier is not soluble in aqueous solution at temperatures of up to 42°C, more preferably of up to 45°C, even more preferably of up to 50°C, yet more preferably of up to 60°C, even more preferably of up to 70°C. Yet more preferably, the solid carrier is not soluble in aqueous solution at temperatures of up to 80°C, even more preferably of up to 85°C, yet more preferably of up to 90°C, even more preferably of up to 95°C, yet more preferably of up to 96°C, even more preferably of up to 97°C. Even more preferred is a solid carrier which is not soluble in aqueous solutions at temperatures of up to 98°C, yet more preferably of up to 99°C. Most preferably, the carrier is not soluble in aqueous solutions at temperatures of up to 100°C. Specifically, the term "solid carrier" in accordance with the present invention does not encompass gaseous, fluid or semi-solid carriers such as, for example, gels that are capable of swelling like hydrogels (e.g. PolyHema) or gelatinous protein mixtures (e.g. Matrigel). Also not encompassed are carriers consisting of agarose. Further, the term does not encompass solubilisable carrier such as, for example, soluble polymers, e.g. polysaccharides, polypeptides or polyethyleneglycols, or crystals. In the context of the present invention, the term "aqueous solution" refers on one hand to water but extends on the other hand also to buffered solutions and hydrophilic solvents miscible with water, thus being able to form a uniform phase. Examples for aqueous solutions comprise, but are not limited to solutions wherein the solvent is water, methanol, ethanol or higher alcohols as well as mixtures thereof. Any solid material may be used as a carrier, such as e.g. polyurethane (PU), polyamide (PA), polyester (PE) and cellulose acetate (CA), glass, polyester, fleece wadding, open-porous foam plastic, reticular plastic and structures derived from marine sponges (porifera). The carrier may consists of a biodegradable material, such as for example, a polyester such as e.g. a polylactide or a polyglycolide, a polyanhydride, a polyurethane, a poly (ester amide), a polyphosphazene, a calcium sulphate, a collagen sponge, a calcium phosphate or a poly(trimethylene carbonate) (Kluin OS, van der Mei HC, Busscher HJ, Neut D., (2013) Expert Opin Drug Deliv., 10(3):341-51.; Zhang Y, Yang J. (2013), J Mater Chem B Mater Biol Med.;1(2):132-148.) or biodegradable magnesium alloys (N. Hort, Y. Huang, D. Fechner, M. Störmer, C. Blawert, F. Witte, C. Vogt, H. Drücker, R. Willumeit, K.U. Kainer, F. Feyerabend, Acta Biomaterialia, (2010) Volume 6 (5), 1714-1725). More preferably, the carrier consists of polyurethane.

To achieve a compact design of the coated affinity-reagent-coupled solid carrier it is preferred that the carrier displays a relatively large surface as compared to its overall measurement. This can be achieved e.g. by using a foam with an open porous structure, a fleece (wadding), a hollow fibre, a woven or nonwoven mesh or a setting using many parallel small tubes or filaments, for example those similar to a porous hollow fibre design currently used in haemodialysis (see e.g. the world wide web at fmc-ag.com/262.htm). It is further preferred that the pore-sizes of the carrier material are compatible with the flow-through of blood. Even more preferably, the pore size of the carrier is between 1.5 and 2 mm and allows the passage of blood in a range from 10 to 5000 mL/min (Tscheliessnig et al. 2012, Materials Today, Volume 15, Issue 9, page 394-404). For example, the carrier may be in the form of a device that is in direct contact with the blood of a patient such as e.g. a tubing set or a different material used in an oxygenator, a blood reservoir, an arterial filter or a part of an extracorporeal circulation device.

The term "at least", as used herein, refers to the specifically recited amount but also to more than the specifically recited amount or number. Therefore the term "at least one affinity-reagent" refers to exactly one affinity-reagent but also to more than one affinity-reagent, such as, for example, at least two, such as at least three, such as at least four etc. as well as to exactly two, exactly three, exactly four, exactly five etc. affinity-reagents being coupled to the carrier. It will be appreciated that the number of affinity-reagents does not refer to the number of individual molecules of one type of affinity-reagent but to the number of different affinity-reagents, i.e. to the number of different types of affinity-reagents that are coupled to the carrier.

The term "affinity-reagent" as used herein refers to a molecule which binds specifically to its target molecule(s). The part of the affinity-reagent that determines the specificity for said target molecule is referred to as "targeting site" herein. It is well known in the art that an affinity-reagent does not have to bind its target molecule in its entirety but instead usually interacts with a defined part thereof. This part of the target molecule is referred to as "binding site" herein. The binding site, which in case of antibody-binding is referred to as an epitope, may, for example, be a particular sequence of amino acids, bases or saccharides. Alternatively, the affinity-reagent(s) may recognise a structural pattern which may be formed by neighbouring or distant units of the target molecule. In both cases, the binding between the affinity-reagent(s) and the target molecule(s) is mediated by non-covalent forces acting between the targeting site of the affinity-reagent(s) and the binding site of the target molecule(s). Well-known pairs of affinity-reagents and corresponding target molecules are, for example, antibody and antigen (binding to each other via their respective epitopes and paratopes), Streptavidin and Biotin, receptor and ligand, such as e.g. Pattern-Recognition Receptors (PRRs) and Pathogen Associated Molecular Patterns (PAMPs), receptor-tyrosine-kinases and their ligands, death receptors and their ligands, etc. Examples of affinity-reagents are well known in the art and include, for example, (poly)peptides, nucleic acid molecules, polysaccharides and organic compounds. (Poly)peptides, nucleic acid molecules, polysaccharides and organic compounds are well known in the art and will be described in further detail herein below.

As used herein the term "binds specifically" indicates that the affinity-reagent(s) bind(s) via its targeting site with high affinity to the binding site in the target molecule. In other words, the affinity-reagent(s) does/do not exhibit cross-reactivity. An affinity-reagent is considered not to exhibit cross-reactivity if it does not bind molecules unless they comprise a portion having at least 65%, such as at least 70%, such as at least 75%, such as at least 80%, such as at least 85%, preferably at least 90% such as at least 95%, such as at least 98% etc. structural identity (as calculated using methods known in the art) to the binding site of the target molecule. Those having skill in the art will know how to determine percent identity between/among molecules using, for example, algorithms such as those based on the NCBI BLAST algorithm (Stephen F. Altschul, Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402), CLUSTALW computer program (Thompson Nucl. Acids Res. 2 (1994), 4673-4680) or FASTA (Pearson and Lipman, Proc. Natl. Acad. Sci., 1988, 85; 2444), as known in the art. The NCBI BLAST algorithm is preferably employed in accordance with this invention for determining sequence identity between nucleic acid sequences or amino acid sequences. The BLASTN program for nucleic acid sequences uses as default a word length (W) of 11, an expectation (E) of 10, M=5, N=4, and a comparison of both strands. For amino acid sequences, the BLASTP program uses as default a word length (W) of 3, and an expectation (E) of 10. The BLOSUM62 scoring matrix (Henikoff, Proc. Natl. Acad. Sci., 1989, 89:10915) uses alignments (B) of 50, expectation (E) of 10, M=5, N=4, and a comparison of both strands. Methods for determining identity between other molecules e.g. polysaccharides or lipids are also well known in the art. Exemplary programs for identifying similar molecules can be found in the world wide web under emolecules.com/ or under pubchem.ncbi.nlm.nih.gov/. Affinity-reagents may, alternatively or additionally, also be described or specified in terms of their binding affinity. Preferred binding affinities include those with a dissociation constant or K_{d} less than 10⁻⁷M, such as less than 5x10⁻⁸M, more preferably less than 10⁻⁸M, such as less than 5x10⁻⁹M, such as less than 10⁻⁹M, such as less than 5x10⁻¹⁰M, such as less than 10⁻¹⁰M, such as less than 5x10⁻¹¹M, such as less than 10⁻¹¹M, such as less than 5x10⁻¹²M, such as less than 10⁻¹²M, such as less than 5x10⁻¹³M, such as less than 10⁻¹³M, such as less than 5x10⁻¹⁴M, such as less than 10⁻¹⁴M, such as less than 5x10⁻¹⁵M, such as less than 10⁻¹⁵M.

As used herein the term "is coupled/coupling" indicates that the affinity-reagent(s) is/are attached to the solid carrier either directly or indirectly. The affinity-reagent(s) may be attached to any accessible surface of the carrier such as the outer surface, the inner surface (where present), or both. It is preferred that the affinity-reagent(s) is/are coupled to those surfaces of the carrier that will be in contact with the sample. Further, the coupling to the surface(s) of the carrier may be effected via a direct binding of the affinity-reagent(s) to said surface(s) of the solid carrier or alternatively, via an indirect binding via a third compound commonly used in the art such as for example via a bi- or multifunctional bridging molecule covering the solid carrier, or via a combination of direct and indirect coupling. Bi- or multifunctional bridging molecules are well known in the art and will be described in detail herein below. Independently of whether the coupling is direct or indirect it can be effected by a binding via a covalent or a non-covalent bond. A covalent bond can be achieved e.g. by a chemical reaction between the affinity-reagent(s) and a carrier material or between a bridging molecule and the affinity-reagent(s). Examples for non-covalent binding comprise weak bonds such as van-der-Waal's bonds or polar bonds.

The term "target molecule" as used herein refers to a molecule that is specifically bound by the affinity-reagent(s), i.e. the molecule containing the binding site that interacts with the targeting site of the affinity-reagent(s) with high affinity. Preferably, the target molecule is a biological target molecule, i.e. a naturally occurring target molecule. The biological target molecule may for example be a (poly)peptide, a nucleic acid molecule, a polysaccharide or a lipid. Preferably, the (biological) target molecule is a molecule that can typically be found in body fluids such as, e.g., blood. To enable interaction with the affinity-reagent(s) it is required that the target molecule and its binding site(s) are accessible. The (biological) target molecule may be a soluble molecule such as, for example, a plasma protein. Alternatively, and preferably, the biological target molecule is expressed on the surface of a cell, such as a target cell, or of a virus. It can, for example, be a membrane protein, i.e. a protein that is inserted into or associated with the cell membrane in such a way that the binding site which is recognised by the affinity-reagent(s) is exposed and accessible from the extracellular space. Non-limiting examples of biological target molecules include cluster of differentiation (CD) molecules, molecules used as differentiation marker to identify e.g. progenitor stem cells or cells from the myeloid lineage, cell-membrane-associated adhesion molecules or cell membrane molecules relevant for signalling. Preferred biological target molecules are, for example, EpCAM, Her2 and Fas (CD95). The term "target cell" as used herein refers to a cell which is to be isolated from a sample such as, e.g., a body fluid, by using the coated affinity-reagent-coupled carrier. In accordance with the invention a target cell therefore is a cell which expresses the biological target molecule and which therefore interacts with the affinity-reagent(s). The term target cell also refers to microorganisms such as for example bacteria. Preferred target cells are circulating tumour cells, endothelial cells, foetal cells, cells of the immune system and precursors thereof. These cells are well known in the art and will be described in more detail herein below. It is preferred that the biological target molecule that is present on the surface of the target cell is expressed on the target cell in a higher amount than on any other cell. Preferably, the amount of the biological target molecule that is expressed on the target cell is at least 1.5-fold the amount expressed on any other cell.

The definition of the term "at least" as provided herein above also applies to this embodiment and includes, for example, at least 2-fold, at least 2.5-fold, at least 3-fold, at least 3.5-fold, at least 4-fold, at least 4.5-fold, at least 5- fold, at least 5.5-fold, at least 6-fold, at least 6.5-fold, at least 7-fold, at least 7.5-fold, such as at least 8-fold, at least 8.5-fold, at least 9-fold, at least 9.5-fold at least 10-fold, at least 20-fold, at least 50-fold, at least 100-fold, at least 1000-fold and so on as well as exactly 1.5-fold, exactly 2-fold, exactly 2.5-fold, exactly 3-fold, exactly 3.5-fold, exactly 4-fold, exactly 4.5-fold, exactly 5-fold, exactly 5.5-fold, exactly 6-fold, exactly 6.5-fold, exactly 7-fold, exactly 7.5-fold, exactly 8-fold, exactly 8.5-fold, exactly 9-fold, exactly 10-fold, exactly 20-fold, exactly 50-fold, exactly 100-fold, exactly 1000-fold and so on.

Preferably, the affinity-reagent recognises a binding site that is unique (1) for the target molecule and/or (2) for the target cell. It will be appreciated that this does not exclude that the biological target molecule or an isoform thereof is expressed on a non-target cell. However, in that case the binding site that is recognised by the affinity-reagent(s) is not accessible in the biological molecule(s) expressed on the non-target cell due to, for example, a different folding or a different post-translational modification such as e.g. a different glycosylation pattern of the biological target molecule. Similarly, if an isoform of the biological target molecule is expressed on (a) non-target cell(s) then the unique binding site recognised by the affinity-reagent(s) is not present or not accessible in that isoform. Isoforms are different forms of the same proteins which may be encoded by closely related gene duplicates or which may arise by single nucleotide polymorphisms or alternative splicing. More preferably, the biological target molecule is only expressed on target cells, but is not expressed on non-target cells. Methods for detecting and determining the relative amount of a biological target molecule that is expressed on a cell are well known in the art and include, for example, fluorescence activated cell sorting (FACS), methods using immunofluorescence, immunohistochemistry, immunocytochemistry, or binding studies to receptors.

In a first step of the method of the invention, the affinity-reagent(s) is/are coupled to the solid carrier by incubating the solid carrier with a composition containing the affinity-reagent(s). The term "incubation" refers to bringing the carrier into contact with the composition comprising the affinity-reagent(s) under conditions that allow the affinity-reagent(s) to attach to the carrier or to an intermediate layer attached thereto. Exemplary incubation conditions include those where incubations are effected from 20 minutes to 12 hours. The temperature range may be from 4°C to 50°C, preferably 20 to 37°C. It is understood that incubation conditions need to be adapted to the affinity-reagent(s) to be attached to the solid carrier. Further, it is known in the art that the optimal incubation time and the optimal incubation temperature are interdependent, i.e. that a certain incubation time may be optimal at a particular temperature but that a longer or shorter incubation time may be suitable at a different temperature. Usually, incubation at a higher temperature is carried out for a shorter time than incubation at a lower temperature. Further, it is known in the art that incubation can be effected by different methods such as, for example, submerging the carrier in a solution comprising the affinity-reagent(s), spraying the carrier with a composition comprising the affinity-reagent(s), inducing a solution comprising the affinity-reagent(s) to flow over the carrier, etc. After incubation, the composition comprising the affinity-reagent(s) is removed. The removal of the composition is understood as a qualitative removal of the composition. The removal may be effected e.g. by suction, e.g. by using a conventional pump to which a pipette may be attached, and the like, compression, blowing or drying. Optionally, such steps are combined. Suitable methods for drying in accordance with the present invention include, without being limiting, air-drying or vacuum-drying. Preferably, the drying step removes the liquid in volume to at least 95 % such as at least 98 % or at least 99 %, 99.5 % or 99.8 %.

Further, the method may optionally comprise a step (ib) of washing the carrier after incubation with the composition comprising the affinity-reagent(s), for example in order to remove affinity-reagent molecules that are not or only loosely attached to the carrier. This optional washing step may be effected using an aqueous solution such as e.g. phosphate buffered saline (PBS) or any other buffered aqueous solution such as, for example, Tris/HCl, MOPS, or HEPES. Such buffered aqueous solutions or buffers may include anorganic salts like sodium chloride, potassium chloride, calcium chloride or magnesium chloride at concentrations from 5 to 500 mM. The washing step may in turn be followed by an optional drying step, as defined herein above.

The general principles of coupling the affinity-reagent(s) to the solid carrier have been described herein above and the preferred coupling methods are described in more detail herein below. Preferably the affinity-reagent(s) is/are coupled to the carrier indirectly via a bridging molecule. For example, the affinity-reagent(s) may be coupled to the carrier (i) via (a) bifunctional bridging molecule(s), (ii) via (a) multifunctional bridging molecule(s), or (iii) via a combination thereof.

The term "bifunctional bridging molecule" as used herein refers to a molecule that has two functional groups and serves as a molecular bridge between the affinity-reagent(s) and the carrier or between the affinity-reagent(s) and a layer coating the carrier such as, for example, a layer comprising a multifunctional bridging molecule such as, for example, polyethyleneimine and/or a methacrylate hydrogel. Bifunctional molecular bridging molecules in this sense are well known in the art and include, for example, succinimidyl-[(N-maleimidopropionamido)-dodecaethyleneglycol] ester (SM(PEG)12) or the same reagents with a different PEG-spacer length n(SM(PEG)n, ethyleneglycolbis [succinimidylsuccinate] (EGS), bis[2-(succinimidoxycarboynyloxy)ethyl]sulfone (BSOCOES), dithiobis[succinimidylpropionate] (DSP), disuccinimidyltartarate (DST), succinimidyl-2-([4,4'-azipentanamido]ethyl)-1,3'-dithioproprionate (SDAD), sulfosuccinimidyl-2-(m-azido-o-nitrobenzamido)-ethly-1,3'-proprionate (Sulfo-SAND), Sulfosuccinimidyl-4-(*N*-maleimidomethyl)cyclohexane-1-carboxylate (Sulfo-SMCC) and silanes. Preferably, the bifunctional bridging molecule is a silane. The term "silane" is used in the context of the invention in line with the definition of the International Union of Pure and Applied Chemistry (IUPAC) and describes a group of compounds consisting of a silicon matrix and hydrogen and/or functional groups. A silane according to this definition may be branched (iso- and neo-silanes) or non-branched (n-silanes). Preferably, the silane is an epoxysilane, an aminosilane, a thiosilane or a methacrylsilane. It is also envisioned that more than one bifunctional bridging molecule is used. If more than one bifunctional bridging molecule is used to couple the affinity-reagent(s) to the carrier or to a layer coating the carrier, the different bifunctional bridging molecules may be applied subsequently or at the same time.

The term "multifunctional bridging molecule" as used herein refers to a molecule that comprises more than two functional groups and serves as a molecular bridge between the affinity-reagent(s) and the carrier or between the affinity-reagent(s) and a layer coating the carrier such as, for example, a layer comprising a bifunctional bridging molecule, such as a silane. Such multifunctional bridging molecules are well known in the art and include, for example, polyhydroxyethyl methacrylate (polyhema or pHEMA), polyglycidoxy methacrylate (pGMA) and polyethyleneimine (PEI). It is also envisioned that more than one multifunctional bridging molecule is used. If more than one multifunctional bridging molecule is used to couple the affinity-reagent(s) to the carrier or to a layer coating the carrier, the different multifunctional bridging molecules may be applied subsequently or at the same time. It is preferred that pHEMA and pGMA, alone or in combination, are employed in the form of a methacrylate hydrogel. A hydrogel is a water-swollen, polymeric structure containing hydrogen bonds bridging networks of polymer chains (Peppas, N. A., Huang, Y., Torres-Lugo, M., Ward, J. H. and Zhang, J., Ann. Rev. Biomed. Eng., 2000, 2, 9-29). Protein adsorption to these networks is modulated by the distribution and organisation of the water within the hydrogel. It has been argued that the water sterically inhibits the adsorption of the protein to the polymer (Holly FJ., J Polym Sci Polym Symp. 1979;66:409}17). As hydrogels have poor mechanical properties, they are mostly used on top of other polymeric substrates. The term "methacrylate hydrogel" refers to such a water-swollen, polymeric structure comprising methacrylate derivatives such as, for example, polyhydroxyethyl methacrylate (polyhema or pHEMA) and/or polyglycidoxy methacrylate (pGMA). Preferably, the methacrylate hydrogel comprises a mixture of pHEMA and pGMA. More preferably, the hydrogel contains equal parts of pHEMA and pGMA. The polymeric structure of pHEMA allows reactions with the solid carrier on the one side and with the affinity-reagent(s) on the other side, the epoxy groups of pGMA are also useful for covalent binding to the carrier on the one hand and the affinity-reagent(s) on the other. Further, the hydrogel increases surface hydrophilicity and therefore the blood-compatibility.

According to possibility (i) a bifunctional bridging molecule is attached to the carrier via one of its two functional groups. The second functional group of the bifunctional bridging molecule is thus available for coupling the affinity-reagent(s) thereto.

Possibility (ii) comprises applying a multifunctional bridging molecule to the carrier before attaching the affinity-reagent(s) to the multifunctional bridging molecule. At least one of the functional groups is used in this case to attach the bridging molecule to the carrier, leaving the remaining functional groups of the multifunctional bridging group available for attaching the affinity-reagent(s) thereto. Preferred multifunctional bridging molecules are polyhydroxyethyl methacrylate (polyhema or pHEMA), polyglycidoxy methacrylate (pGMA) and polyethyleneimine (PEI). These may be used individually or in combination in order to attach the affinity-reagent(s) to the carrier. For example, a mixture comprising polyhydroxyethyl methacrylate and polyglycidoxy methacrylate may be employed, for example, in the form of a hydrogel. Preferably such a mixture comprises equal parts of the two components. Such a mixture provides a stable covalent network in the hydrogel and allows covalently anchoring this intermediate layer to the carrier (or to a layer comprising a bifunctional bridging molecule or PEI which is attached to the carrier) on the one hand and the affinity-reagent(s) on the other. As a further example, coupling of the affinity-reagent(s) to the solid carrier can also be effected via more than one multifunctional bridging molecule such as, for example, PEI and a methacrylate hydrogel. In this case, PEI is attached to the solid carrier before a methacrylate hydrogel is added which interacts with the PEI layer on the one side and with the affinity-reagent(s) on the other. The PEI coating can be carried out under alkaline or acidic conditions. Alkaline coating with PEI results in more stable binding and is therefore preferred. The epoxy groups of pGMA are useful in establishing covalent bonds with the PEI layer on the one hand and the affinity-reagent(s) on the other hand.

Further, the affinity-reagent(s) may be attached to the carrier via a combination of (a) bifunctional and (a) multifunctional bridging molecule(s) (possibility (iii)). For example, the affinity-reagent(s) may be coupled to the carrier via PEI and a bifunctional bridging molecule. When the bifunctional bridging molecule is not a silane, the carrier is first covered with PEI and the bifunctional bridging molecule is attached thereto. The remaining free functional group of the bifunctional bridging molecule is then employed to attach the affinity-reagent(s) thereto. On the other hand, if the bifunctional bridging molecule is a silane, it is preferred that the carrier is first covered with a silane before PEI is attached to the silane-covered carrier. Similar considerations apply with regard to the combination of a methacrylate hydrogel and a bifunctional bridging molecule. When the bifunctional bridging molecule is not a silane, the carrier is first covered with the methacrylate hydrogel and the bifunctional bridging molecule is attached thereto. The remaining free functional group of the bifunctional bridging molecule is then employed to attach the affinity-reagent(s) thereto. On the other hand, if the bifunctional bridging molecule is a silane, it is preferred that the carrier is first covered with a silane before a methacrylate hydrogel is attached to the silane-covered carrier. In the latter case, i.e. if a methacrylate hydrogel and a silane are employed, it is preferred that the methacrylate hydrogel comprises pHEMA. It is further preferred that, when combined with a silane, the methacrylate hydrogel does not comprise pGMA. As can be seen from the above, the order in which the bifunctional and the multifunctional bridging molecule to be employed are used depends on the individual molecules.

In any of the coupling strategies, whenever the coupling is mediated via epoxy groups, coupling efficiency can be further increased by addition of ammonium sulphate, e.g. at a concentration of 1 M. This technique is well known in the art (see e.g. salt-induced immobilisation to epoxide, Wheatly and Schmidt, Journal of Chromatography, 644 (1993), page 11-16).

It is preferred that the density of affinity-reagent molecules coupled to the carrier in this first step of the method allows for the affinity-reagent(s) to bind a maximal amount of target molecules or a maximal number of target cells. Methods for determining an optimal density of affinity-reagent molecules on the carrier are known in the art. For example, carriers with different densities of the affinity-reagent(s) attached thereto can be produced by employing different concentrations of the affinity-reagent(s) during the coupling step. The different carriers can then be assessed for their capability to specifically bind target molecules or cells, e.g. by incubation with a sample containing a defined amount of target molecules or cells. The carrier with the maximal capability of specifically binding target molecules or cells is the carrier with the optimal density of affinity-reagents. In case the maximal capability of specifically binding target molecules or cells is shown by more than one carrier, amongst these carriers the one should be chosen that was produced by incubation with the lowest concentration of the affinity-reagent(s). In this regard, it is understood that the number/amount of affinity-reagent molecules attached to the carrier should be as high as possible in order to provide a maximal number of binding sites for the target molecules or cells while at the same time the distance between affinity-reagent molecules should be chosen such that steric hindrance is avoided. For example, in case the affinity-reagent is an antibody the concentration of the antibody is between 10ng/ml and 400µg/ml, preferably between 100ng/ml and 200µg/ml and more preferably between 1µg and 100µg/ml.

It is further preferred that the coupling is directed, i.e. that the affinity-reagent is coupled to the carrier in a manner that ensures that the targeting site(s) of the affinity-reagent are distant to the carrier, whereas the coupling site of the affinity-reagent is proximal to the carrier. Methods for directed coupling of an affinity-reagent to a carrier are well known in the art and include, for example, the methods described herein above. Depending on the affinity-reagent to be coupled to the carrier, it is known in the art how the methods are to be adjusted, if required, in order to achieve directed coupling. For example, in case the affinity-reagent is an antibody it can be ensured that the antibody is coupled to the carrier via its Fc-part.

In a further step, a compound or a composition is applied to the affinity-reagent-coupled carrier in a manner that results in the formation of a layer, also referred to herein as an overlay, that fully or partially covers the overlaid material. For this purpose the affinity-reagent-coupled carrier is contacted with a compound or a composition as defined below and incubated therewith.

The considerations with regard to the duration and method of the incubation as well as with regard to the temperature at which the incubation is effected as provided with regard to the incubation of the carrier with a composition comprising the affinity-reagent(s) apply *mutatis mutandis* also to the incubation with the composition for overlaying the affinity-reagent. Further, the methods described for removing the composition after incubation and in particular for drying as provided herein above also apply with regard to the steps of overlaying the carrier with further compounds.

The process of applying an overlay is also referred to as "overlaying" or "blocking" herein. Preferably, a first overlay fully or partially covers at least the binding positions on the carrier to which no affinity-reagent is bound. In addition to the binding positions on the carrier to which no affinity-reagent is bound, the affinity-reagent, in particular the parts thereof which are not involved in determining the specificity of the affinity-reagent, may also be covered by the first overlay. As used herein the term "binding positions on the carrier" refers to reactive and accessible sites on the carrier at which unspecific binding of molecules can occur, i.e. to potential sites of unspecific binding. This includes, for example, chemically reactive groups of the carrier material or of the bridging molecule(s) attached thereto. Coupling an affinity-reagent to such binding position renders them inaccessible and therefore unavailable for the unspecific binding of molecules. Therefore, the term "binding positions on the carrier to which no affinity-reagent is bound" refers to binding sites which are not, either directly or indirectly and either covalently or non-covalently, coupled to an affinity-reagent and which therefore remain accessible until they are covered by the first overlay. In accordance with the present invention the term "fully covers" refers to the first overlay rendering all such binding positions inaccessible for the binding of molecules. On the other hand, the term "partially covers" refers to the first overlay rendering some of the binding positions inaccessible for the binding of molecules while leaving the others accessible for the binding of molecules. It is preferred that the first overlay covers at least 30% of the binding positions on the carrier to which no affinity-reagent is coupled, such as at least 40%, such as at least 50%, such as at least 60%, such as at least 70% of the binding positions on the carrier to which no affinity-reagent is coupled. More preferably, the first overlay covers at least 80% of the binding positions on the carrier to which no affinity-reagent is coupled, such as at least 85%, such as at least 90%, such as at least 95%, such as least 99% of the binding positions on the carrier to which no affinity-reagent is coupled. Most preferably, the first overlay covers 100% of the binding positions on the carrier to which no affinity-reagent is coupled, i.e. it fully covers the binding positions on the carrier to which no affinity-reagent is coupled.

Depending on the order in which steps (ii) and (iii) are performed, the term "first overlay" as used herein refers to a deposit of a composition comprising heparin and/or a functional derivative thereof in accordance with (ii) or to a deposit of a composition comprising stabilising excipients in accordance with (iii). In other words, the "first overlay" is always the first layer on top of the affinity-reagent, i.e. if the affinity-reagent-coupled carrier is first overlaid with a composition comprising heparin and/or a functional derivative thereof in accordance with (ii) than the first overlay is the overlay comprising heparin and/or a functional derivative thereof. If, on the other hand, the affinity-reagent-coupled carrier is first overlaid with a composition comprising stabilising excipients in accordance with (iii) then the overlay comprising stabilising excipients is referred to as the "first overlay".

In accordance with the present invention, one of the overlays comprises heparin and/or a functional derivative thereof.

The term "heparin" refers to members of the glycosaminoglycan family of polysaccharides which are composed of repeats of a variably sulphated disaccharide unit. This disaccharide unit consists of D-glucosamine and an uronic acid (D-glucuronic acid or L-iduronic acid) which can be sulphated at oxygen or nitrogen atoms. The most common disaccharide unit is composed of a 2-O-sulfated iduronic acid and 6-O-sulfated, N-sulfated glucosamine, IdoA(2S)-GlcNS(6S). The number of saccharide units comprised in a heparin molecule and therefore its molecular weight may vary. However, most heparins have a molar mass between 4 and 40kDa. Depending on the number of monosaccharide units and on molecular weight, heparin is referred to as unfractioned heparin (UFH, having more than 17 monosaccharide units and a molecular weight of approximately 6 to 30kDa) or as fractioned or low molecular weight heparins (LMWH, having between 5 and 17 monosaccharide units and a molecular weight of less than 6kDa). As used herein the term "heparin" also encompasses heparin salts. So far, the best known function of heparin is its ability to inhibit the clotting cascade which renders it suitable for therapeutic purposes, e.g., in preventing or treating thrombosis. In addition, in accordance with the present invention, a further ability of heparin, namely to inhibit unspecific binding, is employed. Heparins may, for example, be extracted from natural sources, such as e.g. porcine intestinal mucosa or from bovine lung or can be obtained commercially, e.g. from Fresenius AG; V+P Medic-Clean; Ratiopharm; Sigma-Aldrich or Novartis.

The term "functional derivative thereof" refers to a molecule which is structurally similar to heparin and which retains the ability of heparin to reduce or prevent the unspecific binding of molecules or cells to an affinity-reagent-coupled solid carrier. Non-limiting examples for functional heparin derivatives include pentosan polysulfate, heparansulfate and fondaparinux. As a further example, danaparoid, a mixture of heparansulphate, dermatane sulphate and chondroitin-4- and 6-sulphate, may be employed. Heparin derivatives can be isolated from natural sources or can be produced semi-synthetically (Fernández et al., 2006).

For a heparin derivative to be considered as functional in accordance with the present invention it is not required that it retains the anti-coagulant activity of heparin as long as it retains the ability of heparin to reduce or prevent the unspecific binding of molecules or cells to an affinity-reagent-coupled solid carrier. It is preferred that the functional heparin derivative retains at least 40%, such as at least 50%, such as at least 60%, such as at least 70%, such as at least 80%, such as at least 90%, such as at least 95% of the activity of heparin in reducing or preventing the unspecific binding of molecules or cells. More preferably, the functional heparin derivative exhibits at least the same activity as heparin in reducing or preventing unspecific binding of molecules or cells. In order to assess whether a heparin derivative retains the ability of heparin to reduce or prevent the unspecific binding of molecules or cells to an affinity-reagent-coupled solid carrier, two (or more) affinity-reagent-coupled carriers can be overlaid (i) with the heparin derivative and (ii) with heparin, respectively and the unspecific binding to the differentially coated carriers can be assessed and compared. In order to determine how much of the activity of heparin in reducing the unspecific binding of non-target molecules or non-target cells is retained by a heparin derivative, e.g. in order to determine whether a heparin derivative is a functional heparin derivative as defined herein, the methods described herein below may be employed.

The "composition comprising heparin and/or a functional derivative thereof" of the present invention comprises at least one of the recited compounds. Also encompassed by this term are compositions consisting of heparin and/or a functional derivative thereof.

The term "comprising heparin and/or a functional derivative thereof" encompasses that the composition may further contain other substances such as, e.g., water, buffer, other solvents and/or specific additives which are further described herein below as well as, additional known blocking agents such as e.g. human serum albumin, bovine serum albumin or methoxy terminated polyethyleneglycol (mPEG). It is preferred that the composition contains heparin and/or a functional derivative thereof as the only blocking agent(s). Alternatively, depending on other parameters such as e.g. the coupling method the presence of an additional blocking agent may be preferred. For example, if maleimide chemistry is used for coupling it is preferred that the composition comprising heparin and/or a functional derivative thereof also comprises mPEG-Thiol. The composition may, optionally, comprise further molecules capable of altering the characteristics of heparin and/or a functional derivative thereof thereby, for example, increasing, stabilising and/or modulating their function. Preferably, the composition is an aqueous solution, i.e. the solvent is water.

In order to ensure that a first overlay comprising heparin and/or a functional derivative thereof fully or partially covers at least the binding positions on the carrier to which no affinity-reagent is bound as defined herein above, the composition comprising heparin and/or a functional derivative thereof needs to comprise a sufficient amount of the recited compounds. It can easily be established how much heparin and/or a functional derivative thereof needs to be comprised in the composition in order to achieve full or partial coverage of at least the binding positions on the carrier to which no affinity-reagent is bound. It is preferred that the composition comprises heparin in a concentration of at least 0.5 mg/mL, such as at least 1 mg/mL, such as at least 2 mg/mL, such as at least 3 mg/mL, such as at least 4 mg/mL, such as at least 5 mg/mL, such as at least 6 mg/mL, such as at least 7 mg/mL, such as at least 8 mg/mL, such as at least 9 mg/mL, such as at least 10 mg/mL (in case of ammonium heparin purchased from Sigma Aldrich 1 mg presently corresponds to 140 international units). More preferably, the composition comprises heparin in a concentration of at least 5 mg/ml. For the functional heparin derivative, concentrations that correspond to the concentrations indicated for heparin above are preferred. In this context it will be appreciated that the preferred concentration (mg/mL) may differ from the values indicated for heparin depending on the molecular weight of the functional derivative. The corresponding concentrations on the basis of the molecular weight of the heparin derivative can be calculated without further ado. It will be appreciated that in case the composition is a solution the concentration of heparin and/or a functional derivative thereof should not exceed the maximal solubility of the respective compound in the employed solvent.

With regard to the resulting first overlay, it is preferred that the first overlay comprises heparin at a concentration of at least 0.1 µg per cm² of the surface of the carrier, such as at least 0.5 µg/cm², such as at least 1 µg/cm², such as at least 1.5 µg/cm², such as at least 2 µg/cm², such as at least 2.5 µg/cm², such as at least 3 µg/cm², such as at least 4 µg/cm², such as at least 4.5 µg/cm², such as at least 5 µg/cm², such as at least 5.5 µg/cm², such as at least 6 µg/cm², such as at least 6.5 µg/cm², such as at least 7 µg/cm², such as at least 7.5 µg/cm², such as at least 8 µg/cm², as at least 8.5 µg/cm², such as at least 9 µg/cm², such as at least 9.5 µg/cm², such as at least 10 µg/cm². More preferably, heparin is comprised in the first overlay at a concentration of at least 20 µg per cm² of the surface of the carrier, such as at least 30 µg/cm², such as at least 40 µg/cm², such as at least 50 µg/cm², such as at least 60 µg/cm², such as at least 70 µg/cm², such as at least 80 µg/cm², such as at least 90 µg/cm², such as at least 100 µg/cm². Yet more preferably, heparin is comprised in the first overlay at a concentration of at least 200 µg per cm² of the surface of the carrier, such as at least 300 µg/cm², such as at least 400 µg/cm², such as at least 500 µg/cm², such as at least 600 µg/cm², such as at least 700 µg/cm², such as at least 800 µg/cm², such as at least 900 µg/cm², such as at least 1000 µg/cm². Most preferably, heparin is comprised in the first overlay at a concentration of at least 600 µg/cm². As detailed above, the corresponding concentrations for a functional heparin derivative with a different molecular weight than heparin can easily be calculated.

The thickness of the resulting first overlay can be adjusted depending on the affinity-reagent employed. This can be advantageous in those cases where unspecific binding occurs at the parts of the affinity-reagent that are not specificity-determining, i.e. parts of the affinity-reagent other than the targeting sites. Thus, the thickness of the first overlay may ensure that these parts of the affinity-reagent are covered, whereas the targeting sites of the affinity-reagent remain outside the first overlay. It is preferred that the affinity-reagent is completely covered by the first overlay, i.e. that also the targeting sites of the affinity-reagent are covered by the first overlay. The size of specific affinity-reagents is known in the art and the thickness of the first overlay can be adjusted according to the size of the affinity-reagent to be used without further ado. It is preferred that the first overlay comprising heparin and/or a functional derivative thereof has a thickness in the range of 10 nm to 10 µm. The thickness of the first overlay can be determined by methods well known in the art such as e.g. by X-ray reflectivity (XRR) (Tscheliessnig et al. 2012, Materials Today, Volume 15, Issue 9, page 394-404).

Preferably, the overlay comprising heparin and/or a functional derivative thereof forms a solid coating layer. Accordingly, the first overlay may be generated by incubating the affinity-reagent-coupled carrier with a solution comprising heparin and/or a functional derivative thereof and a subsequent drying step. The overlay does not have to be continuous in order to be referred to as a layer. Instead, patches of the same substance which are present on the same substrate, optionally without being connected to each other, can be referred to as a layer herein.

The step of applying a first overlay comprising heparin and/or a functional derivative thereof in accordance with option (ii) may be followed by an optional washing step (iib), optionally in combination with a subsequent drying step, as defined above. In particular, a washing step is used if the overlay is bound to the targeting sites of the affinity-reagent with more than a weak or moderate on-rate. In that case, the washing step is used to render the targeting sites of the affinity-reagent accessible. However, it needs to be ensured that the overlay is not removed completely, i.e. that the overlay is not removed from the parts of the affinity-reagent and the carrier that may mediate unspecific binding. Therefore, the washing step needs to be effected under mild conditions. For example, the optional washing step may be effected using an aqueous solution such as e.g. phosphate buffered saline (PBS) or any other buffered aqueous solution such as, for example, Tris/HCl, MOPS, HEPES, or other common biochemical buffers, which may include anorganic salts like sodium chloride, potassium chloride, calcium chloride or magnesium chloride at concentrations from 5 to 500 mM. Preferably, the washing step is performed at a pH between 5 and 9, preferably between 6 and 8, for a short time, such as e.g. for between 1 and 30 minutes, preferably for between 1 and 10 minutes, at low temperature such as e.g. at a temperature between 0°C and 37°C, preferably between 4°C and 25°C and using a washing solution which has a low salt content (between 1mM and 1 M). More preferably, the overlaying step is followed by a drying step without a previous washing step. More preferably, the drying step is effected by air-drying.

In another step, the affinity-reagent-coupled carrier is incubated with a composition comprising stabilising excipients.

As used herein, "stabilising excipients" are substances that increase the physical and/or chemical stability of the affinity-reagent(s) which is/are coupled to the solid carrier. Excipients are often used to slow down or prevent physical destabilisation processes such as, for example, protein aggregation or denaturation. Stabilisation can be achieved, e.g., by strengthening of the protein-stabilising forces or by destabilisation of the denatured state. Frequently used protein stabilisers include sugars and polyols, amino acids, dipeptides, tripeptides, amines, saponines, salts, polymers and surfactants or combinations thereof, each of which may exert different stabilising effects. Preferred excipients for the stabilisation of the at least one affinity-reagent are amino acids, peptides, saponines, chitosane and/or carnosine. More preferred is a combination of at least three amino acids or of two amino acids and a saponine. These excipients are well known in the art and are described in further detail herein below.

The incubation with a composition comprising stabilising excipients results in the formation of an overlay. In those cases where the affinity-reagent coupled carrier was previously overlaid by incubation with a composition comprising heparin and/or a functional derivative thereof, said overlay comprising heparin and/or a functional derivative thereof is referred to herein as the first overlay and the overlay resulting from the subsequent incubation with a composition comprising stabilising excipients is referred to herein as the second overlay. In that case, the term "second overlay" refers to a deposit of a composition comprising stabilising excipients. If the order of steps (ii) and (iii) is reversed, i.e. if the affinity-reagent-coupled carrier is first overlaid by incubation with a composition comprising stabilising excipients and subsequently further overlaid by incubation with a composition comprising heparin and/or a derivative thereof, then the term "first overlay" refers to the overlay comprising stabilising excipients and the term "second overlay" refers to the overlay comprising heparin and/or a functional derivative thereof. Independently of the order in which steps (ii) and (iii) are effected, the second overlay is separated from the carrier and/or the affinity-reagent(s) coupled thereto by the first overlay at sites where both overlays are present. However, the second overlay may also be in direct contact with the carrier and/or the affinity-reagent(s) coupled thereto at sites where these are not covered by the first overlay or if first and second overlay are combined into one overlay. Preferably, the second overlay fully or partially covers the parts of the carrier and the affinity-reagent(s) coupled thereto remaining accessible after the first overlaying, if present. In particular it is preferred that the overlay comprising stabilizing excipients fully covers the parts of the carrier and the affinity-reagent(s) coupled thereto remaining susceptible to damages induced by adverse conditions such as e.g. high temperatures, oxidizing conditions and/or irradiation after the first overlaying, if present. The first and the second overlay are not necessarily separate, i.e. the components of the first and the second overlay may be comprised in the same overlay. This can be achieved by applying the two overlays at the same time, i.e. by performing the steps (ii) and (iii) of the method of the invention simultaneously as defined below. In that case, the combined first and second overlays cover the binding positions on the carrier to which no affinity-reagent is bound as well as the affinity-reagent(s) coupled to the carrier. Preferably, they fully cover the binding positions on the carrier to which no affinity-reagent is bound as well as the affinity-reagent(s) coupled to the carrier. However, it is preferred that the components of the first and the second overlay are comprised in separate overlays. This is achieved by coating the affinity-reagent-coupled carrier with a first overlay and subsequently applying the second overlay.

As defined herein above with regard to the overlay comprising heparin and/or a functional derivative thereof, the term "comprises" indicates also with regard to the overlay comprising stabilising excipients that other substances such as, e.g., water, buffer, other solvents may be present in the layer. In a preferred embodiment, the overlay comprising stabilising excipients further comprises albumin, such as e.g. bovine or human serum albumin. It is further preferred that the composition comprising stabilising excipients is a solution. The solution containing the stabilising excipients can be an aqueous or a non-aqueous solution. Non-aqueous solutions are solutions based on a non-aqueous solvent such as, for example, dimethylsulfoxide (DMSO), ethylbenzene, and other polar solvents. Preferably, the composition comprising stabilising excipients is an aqueous solution, as defined herein above. Preferably, the incubation with a composition comprising stabilising excipients is followed by a drying step (iiib). It is further preferred that the drying is effected by air-drying. Further, it is preferred that the incubation with a composition comprising stabilising excipients is not followed by a washing step. If nevertheless a washing step is performed it needs to be ensured, by selecting appropriate washing conditions as defined above, that the overlay comprising stabilising excipients is not removed.

The term "steps (ii) and (iii) are carried out subsequently or simultaneously" indicates that steps (ii) and (iii) may be performed in the described order, in a different order or at the same time. If steps (ii) and (iii) are performed simultaneously heparin and/or a functional derivative thereof and the stabilising excipients may be applied as separate compositions or as a combined composition comprising both heparin and/or a functional derivative thereof and the stabilising excipients. However, it is preferred that steps (ii) and (iii) are performed in the described order, i.e. step (iii) is performed subsequently to step (ii).

The method may further include an optional step (iv) of sterilising the produced carrier. The term "sterilising" refers to a process wherein all live organisms are killed. Methods of sterilisation are well known in the art and include, for example, sterilisation by ethylene oxide (EO), by beta irradiation, by gamma irradiation or by plasma sterilisation. Preferably, the sterilisation is effected by irradiation or EO.

An optional washing step (ivb) may be included in the production method after step (iv) of sterilising the carrier. This washing step is to be performed under sterile conditions. Preferably, such a washing step is performed only if the carrier is to be used immediately but not if the carrier is to be stored before use. If the carrier is washed, the washing step is performed under mild conditions such as e.g. at a pH between 5 and 9, preferably between 6 and 8, for a short time, such as e.g. for between 1 and 30 minutes, preferably for between 1 and 10 minutes, at low temperature such as e.g. at a temperature between 0°C and 37°C, preferably between 4°C and 25°C and using a washing solution which has a low salt content (between 1mM and 1 M) in order to ensure that heparin and/or a functional derivative thereof is not washed off. It is known in the art how to identify further mild washing conditions in order to ensure that the overlay comprising heparin and/or a derivative thereof is not washed off. Preferably, a washing step is only performed if step (ii) was performed before step (iii). It is particularly preferred that after sterilisation no washing step is performed.

The carrier produced by the method of the invention may be stored in liquid or under dry conditions. In case the carrier produced by the method of the invention is to be stored in liquid, the liquid in which the carrier is to be stored comprises stabilising excipients, preferably the stabilising excipients used in the preparation of the carrier. Storage under dry conditions is preferred.

The produced carrier shows reduced unspecific binding of molecules or cells as compared to the same solid carrier to which the same at least one affinity-reagent is coupled but which does not comprise the overlay(s) comprising heparin and/or a derivative thereof, and stabilising excipients. The term "unspecific binding of molecules or cells" refers to any binding not mediated by the targeting site of the affinity-reagent(s). In other words it refers to any binding of molecules that is mediated e.g. by the solid carrier material itself and/or by parts of the affinity-reagent(s) other than the targeting sites. In particular, unspecific binding includes the binding of molecules or cells that do not comprise the specific binding site recognised by the affinity-reagent(s) but that nevertheless bind to the affinity-reagent-coupled carrier (these molecules and cells are also referred to herein as non-target molecules or non-target cells, respectively). Such non-target molecules include, for example, DNA molecules, RNA molecules, polysaccharides, lipids or proteins, i.e. molecules that can typically be found in mixtures for use in methods of affinity purification such as body fluids, for example blood.

Methods for determining unspecific binding are well known in the art and include, for example, the following approach. To determine unspecific binding to a carrier, the following steps can be taken:
(i) incubating a carrier to which an affinity-reagent is coupled (referred to herein as carrier 1) with a sample comprising target and non-target molecules and/or cells under conditions that allow the binding of target molecules or cells to the affinity-reagent-coupled carrier;
(ii) removing the sample and washing the carrier to remove molecules/cells bound with low affinity or not bound,
(iii) determining the amount of molecules and/or the number of cells bound to carrier 1;
(iv) repeating steps (i) to (iii) with a carrier (carrier 2) that is identical to carrier 1 apart from the fact that an affinity-reagent is coupled thereto which differs from the affinity-reagent coupled to carrier 1 only in specificity-determining parts and which does not specifically bind to any molecule that is present and accessible in the sample (also referred to herein as "control affinity-reagent");
(v) subtracting the amount of molecules and/or the number of cells bound to carrier 2 as determined in step (iv) from the amount of molecules and/or the number of cells bound to carrier 1 as determined in step (iii) to obtain the amount of molecules and/or the number of cells specifically bound to carrier 1 (binding to carrier 1 - binding to carrier 2 = specific binding to carrier 1);
(vi) subtracting the amount of molecules or the number of cells specifically bound to carrier 1 as determined in step (v) from the amount of molecules or the number of cells bound to carrier 1 as determined in step (iii) to obtain the amount of molecules and/or the number of cells unspecifically bound to carrier 1 (binding to carrier 1 - specific binding to carrier 1 = unspecific binding to carrier 1).

A "control affinity-reagent" is an affinity-reagent that does not specifically bind to any molecule that is present and accessible in the sample. An accessible molecule is a molecule that is exposed and that is therefore available for binding by the affinity-reagent(s). This excludes, for example, intracellular molecules that are shielded by cell walls or cell membranes from the sample. Preferably an accessible molecule is a molecule on the surface of a cell. It will be appreciated that more than one control affinity-reagent may be used especially in cases where more than one affinity-reagent is present on the carrier to be tested for unspecific binding thereto. In general, for each affinity-reagent a corresponding control affinity-reagent should be used. It will be appreciated, however, that if two or more affinity-reagents differ only in their specificity-determining parts, i.e. in the targeting sites, and are otherwise identical, a single control affinity-reagent may be used as the control for all these affinity-reagents. For example, if several antibodies of the same isotype are used as affinity-reagents a single control antibody of the same isotype may be used as the control affinity-reagent. A carrier which is identical to the carrier to be tested, apart from the fact that that a control affinity-reagent is coupled thereto, is referred to herein as a "control carrier".

It will be appreciated that the washing of the carrier in step (ii), and repetitions thereof, is optional and that repetition of steps (i) to (iii) for carrier 2 does not have to take place after these steps have been performed for carrier 1 but may be performed at the same time. Further, it will be appreciated that the terms "carrier 1" and "carrier 2" do not necessarily refer to a single carrier, respectively. Instead, multiple carriers with the described properties can be employed in performing the above described method. In that case the average of all results obtained for all carriers of type 1 (carrier 1) and all carriers of type 2 (carrier 2) are determined, respectively, before step (v). Accordingly, in step (v) the average values for the carriers are employed to calculate the specific binding to carrier 1 (average binding to carrier 1 - average binding to carrier 2 = specific binding to carrier 1).

In order to determine by how much a heparin overlay reduces unspecific binding as compared to a non-coated carrier, steps (i) to (vi) can be carried out for a heparin-coated carrier (carrier 1) (steps (Ai) to (Avi)) and for a non-coated carrier (carrier 3) (steps (Bi) to (Bvi)). In this method, carriers 1 and 3 are identical apart from the overlay, the control affinity-reagent-coupled carriers 2 and 4 are used in steps (Aiv) and (Biv), respectively, i.e. carrier to is used as a control for carrier 1 in order to determine the unspecific binding to carrier 1 and similarly carrier 4 is used as a control for carrier 3. The following additional steps are performed:
(vii) dividing the unspecific binding to carrier 1 as determined in step (Avi) by the unspecific binding to carrier 3 as determined in step (Bvi) and multiplying the result by 100 to determine the percentage of unspecific binding to carrier 1 as compared to carrier 3; and
(viii) subtracting the result obtained in step (vii) from 100 to determine by how many percent the heparin overlay of carrier 1 reduces the unspecific binding as compared to non-coated carrier 3.

In this regard, it will be appreciated, that steps (Ai) to (Avi) can be, but need not be, carried out at the same time as steps (Bi) to (Bvi) and that carriers 1 to 4 can be incubated with the same sample at the same time or with identical samples (either in parallel or at different times).

In order to determine by how much the heparin overlay reduces unspecific binding as compared to other blocking agents the same method as described herein above for the comparison between a heparin-coated and a non-coated carrier can be employed. In that case carrier 1 is the same heparin-coated carrier as described above, whereas carrier 3 is an affinity-reagent-coupled carrier identical to carrier 1 but coated with the blocking agent that is to be compared to heparin.

A similar approach can be taken in determining how much of the activity of heparin in reducing the unspecific binding of non-target molecules or non-target cells is retained by a heparin derivative, e.g. in order to determine whether a heparin derivative is a functional heparin derivative as defined herein above. In this case, carrier 1 is an affinity-reagent-coupled carrier coated with a functional heparin derivative, carrier 3 is an affinity-reagent-coupled carrier identical to carrier 1 but coated with heparin and carriers 2 and 4 differ from carrier 1 and 3, respectively, in that they are coupled to a control affinity-reagent rather than to the specific affinity-reagent(s) coupled to carriers 1 and 3, respectively. Using these carriers the steps described above are performed and the value obtained in step (vii) indicates the percentage of the activity of heparin in reducing unspecific binding that is retained by the functional heparin derivative.

The unspecific binding to a carrier may be assessed after the first overlay has been applied or after the production process has been completed, i.e. after the further overlay(s) have been applied. Preferably, the unspecific binding to the carrier is assessed after at least the overlay comprising heparin and/or a functional derivative thereof has been applied. In any case the control carrier(s) need(s) to have been treated in the same manner as the carrier to be tested. Accordingly, if the carrier is tested after applying the second overlay, the control carrier also needs to comprise said second overlay. Similar considerations apply if the carrier is tested after a further overlay has been applied. In that case the control carrier also needs to comprise said further overlay. Similarly, if the carrier is tested after it has been washed after the overlaying steps the control carrier needs to have been treated in the same manner.

The sample to be used in assessing unspecific binding of molecules or cells to a carrier may be an artificially produced or a naturally occurring mixture of target and non-target molecules and/or cells. Preferably, the sample is liquid. For example, the sample may be a buffered aqueous solution to which target and non-target molecules/cells have been added or a sample derived from a body fluid such as blood. The term "buffered aqueous solution" refers to a water-based solution that contains substances that keep the pH-value as well as the salt content of the solution in a defined range. Preferably, the properties of the buffered aqueous solution, e.g. the pH-value and the ionic strength, prevent unfavourable changes to the structure and/or function of biological molecules and allow, where required, the survival of cells, preferably mammalian cells, in the solution. It will be understood that the stability of biological molecules and the survival of cells do not need to be maintained for an unlimited amount of time. Instead, it is sufficient, that the molecules are stable and the cells survive in the buffered aqueous solution for a reasonable amount of time, at least the time needed to isolate them from the sample.

Methods for determining the amount of molecules or the number of cells bound to a carrier (as performed e.g. in step (iii) of the above described method) can be performed on the carrier or by eluting the molecules/cells bound to the carrier and analysing the eluate. Such methods are well known in the art and include, for example, determining the amount/number of molecules/cells e.g. by microscopy or by using a cell counting chamber, biochemical assays such as e.g. assays for determining protein content (e.g. the Biuret method, the Lowry method or the Bradford assay), photometric methods, fluorescence activated cell sorting (FACS) or enzyme-linked immunosorbent assays (ELISA). Alternatively, the amount of molecules or the number of cells bound to a carrier can also be determined indirectly, by incubating the carrier with a sample containing a known amount of molecules or a known number of cells and subsequently assessing the amount of molecules or the number of cells remaining in the sample after incubation with the carrier. In case the sample is blood, the number of the different types of blood cells present in the sample before and after incubation with an affinity-reagent-coupled carrier may, for example, be determined using a haemocytometer.

Alternatively, in order to characterise different carriers, the binding kinetics, the on rate (kₐ), the dissociation rate (k_{d}) and the binding constant (K_{D}) for the differentially coated carriers can be determined in a binding assay using labelled target molecules, for example, by surface plasmon resonance spectroscopy (e.g., the biacore system). Labelled target molecules can be produced by methods well known in the art, for example, target proteins may be produced recombinantly, e.g. by introducing a plasmid encoding the target protein and optionally the label into host cells, inducing expression and isolating the target protein by standard techniques such as e.g. affinity chromatography. In case the label is not encoded by the plasmid and expressed together with the target protein, it may be added to the protein after isolation. Suitable labels are well known in the art and include, for example, fluorescence labels, labels which are bound with high affinity by a corresponding affinity-reagent such as e.g. biotin, poly-histidine etc. and labels which have enzymatic activity and which can be detected via their ability to catalyse a, preferably colorimetric, reaction upon addition of their substrate such as e.g. horseradish peroxidase (HRP). It is understood that the labelling procedure, i.e. the conditions under which labelling is carried out, whether a label is added during or after production of the target molecule, etc., depends on the type of target molecule and on the type of label. For example, a peptide tag, such as poly-histidine will usually be added during the production of recombinant a target protein, whereas, for example, biotin can be added to a recombinant protein subsequently to its production, for example by *in-vitro* biotinylation.

Whereas not always necessary, the use of the full-length target molecule in determining the binding to different carriers is preferred. However, the part of the molecule that comprises the binding site, i.e. the site specifically bound by the affinity-reagent(s), may also be sufficient. For example, a peptide that comprises the binding site and a label can be employed. Such peptides need to be soluble and can, for example, be produced synthetically by methods well known in the art such as Fmoc chemistry. In order to determine the binding constant, the coated affinity-reagent-coupled carrier can be probed with different amounts of labelled target molecule which may in turn be detected via their label. The K_{D} can be determined by such measurements, for example by fitting the data to a 1:1 Langmuir binding model.

In accordance with the invention the unspecific binding of molecules or cells is reduced, i.e. a decreased amount or number of non-target molecules or non-target cells is bound to the coated affinity-reagent-coupled solid carrier after incubation with a sample, such as a body fluid. Preferably, the amount or number of non-target molecules or non-target cells that is bound to the coated affinity-reagent-coupled solid carrier is reduced by at least 20%, such as at least 30%, such as at least 40% as compared to the amount or number of non-target molecules or non-target cells that is bound to the same solid carrier to which the same affinity-reagent(s) is/are coupled but which does not comprise the overlay(s) comprising heparin and/or a derivative thereof and stabilising excipients. More preferably, the amount or number of non-target molecules or non-target cells that is bound to the coated affinity-reagent-coupled solid carrier is reduced by at least 50%, such as at least 60%, such as at least 70%, such as at least 80%, such as at least 90%, such as at least 95% as compared to the amount or number of non-target molecules or non-target cells that is bound to the same solid carrier to which the same affinity-reagent(s) is/are coupled but which does not comprise the overlay(s) comprising heparin and/or a derivative thereof. Preferably, the amount or number of non-target molecules or non-target cells that is bound to the coated affinity-reagent-coupled solid carrier is reduced by 100%, i.e. the binding of non-target molecules or non-target cells to the coated affinity-reagent-coupled solid carrier is prevented completely. Methods for determining by how much an overlay reduces unspecific binding are well known in the art and include, for example, the methods described herein above.

The term "the same solid carrier to which the same at least one affinity-reagent is coupled but which does not comprise the overlay(s) comprising heparin and/or a derivative thereof' as used herein on the one hand refers to a device consisting of a solid carrier to which the same affinity-reagent(s) is/are coupled, wherein the carrier and the affinity-reagent(s) are not covered by (an) overlay(s) comprising the specified compounds. An affinity-reagent-coupled carrier not comprising the overlay(s) comprising heparin and/or a derivative thereof may either not comprise any overlay at all or may comprise (an) overlay(s) comprising a known blocking agent/mixture of known blocking agents, which is different from/does not comprise heparin and/or a functional derivative thereof and which is usually employed in the art. Known blocking agents include, for example, human serum albumin (HSA), bovine serum albumin, and methoxy terminated polyethyleneglycol (mPEG). More preferably, said known blocking agent is human serum albumin. In order to determine the effect of a particular overlay or of a particular combination of overlays, it is usually required that the non-coated carrier used in a comparison is, apart from lacking an overlay or comprising (a) different overlay(s), identical to the coated affinity-reagent-coupled carrier. Preferably, the affinity-reagent-coupled carrier used as a reference, i.e. the carrier that is not coated with heparin and/or a functional derivative thereof, is an affinity-reagent-coupled carrier coated with HSA. More preferably, the amount of HSA used in coating said reference carrier is the same or up to 10-times higher in weight than the amount of heparin and/or a functional derivative thereof used in overlaying the affinity-reagent-coupled carrier that is coated with heparin and/or a functional derivative thereof. In other words, the carrier produced by the method of the invention preferably provides reduced unspecific binding as compared to such a reference carrier coated with the same or an up to 10-times higher amount of HSA.

In accordance with the present invention it was surprisingly found that the use of a composition comprising heparin and/or a functional derivative thereof for overlaying an affinity-reagent-coupled carrier results in the production of an affinity-reagent-coupled carrier which exhibits a particularly low unspecific binding of non-target molecules and non-target cells. Without wishing to be bound by theory, the inventors hypothesise that unspecific binding often occurs adjacent to the targeting sites of the affinity-reagent(s) and thus, reduces the number of targeting sites that are available for specific binding due to steric hindrance. Further, it is hypothesised that the overlay(s), in particular the overlay comprising stabilising excipients, cover(s) the targeting site of the affinity-reagent with a weak or moderate on-rate. In order to bind to the targeting site of the affinity-reagent, a molecule therefore needs to compete with the overlay(s). This results in enhanced binding specificity. In other words, only molecules that bind to the affinity-reagent with sufficient affinity, i.e. target molecules that are specifically bound by the affinity-reagent, can displace the overlay(s). On the other hand, target molecules that would be unspecifically bound in the absence of the overlay(s) cannot displace the overlay(s) and can therefore no longer bind to the affinity-reagent-coupled carrier. A reduction in unspecific binding leads to an increase in the number of available targeting sites and, consequently, in the amount of target molecules or the number of target cells that are specifically bound to the affinity-reagent-coupled carrier. At the same time the purity of target molecules or target cells isolated by using the affinity-reagent-coupled carrier in accordance with the invention is increased as compared to isolations of the same target molecules or target cells with uncoated affinity-reagent-coupled carriers or with affinity-reagent-coupled carriers coated with blocking substances known in the art. In other words, a binding matrix is provided that allows specifically binding a specific cell type, such as CTCs, from a sample while at the same time preventing the unspecific binding of other cells or molecules. Such other cells may include non-harmful cells, which may even fulfil an important physiological function and/or may be required for the recovery of the patient. Unspecific binding of these cells may lead to a reduction or a depletion of these beneficial cells from the peripheral blood of a patient during apheresis. By reducing unspecific binding, this unintended and potentially harmful side effect is also reduced or prevented. The enhanced purity of target cells isolated by using affinity-reagent-coupled carriers in accordance with the present invention also allows the further characterisation of isolated cells by phenotyping or genotyping. The availability of cell types, including rare cells such as CTCs in a purity that allows and facilitates their further characterisation will be useful for the investigation and scientific understanding of biological processes such as, for example, the different steps of metastasis formation. Further, the analysis of such cells e.g. by phenotyping and genotyping will be of diagnostic, prognostic and potentially predictive value in patients with a variety of diseases or conditions and especially in patients suffering from cancer. Yet another advantage of being able to obtain cells in sufficient amounts and high purity is that they may be used in therapeutic applications such as, for example, adoptive immune transfer or immunisations. Also advantageous is that by including an overlay comprising stabilising excipients, the invention provides a carrier that is less prone to loss of binding activity or affinity of the affinity-reagent(s) during preparation and storage of the carrier. In particular, this allows the sterilisation of the carrier to which the affinity-reagent(s) is/are already coupled and thus the time and expense associated with the production of a carrier under aseptic conditions can be saved and the carrier can nevertheless be employed in applications requiring its sterility.

Thus, in accordance with the present invention, a method was developed that allows the production of an improved sterilisable affinity-reagent-coupled carrier that can be employed in improving the purity of target molecules or target cells which are isolated from a sample by affinity purification. As discussed, this is associated with many advantages for the scientific understanding of biological processes, as well as for therapeutic, diagnostic and prognostic applications and may prove useful in predicting the response to a specific treatment, i.e. in predictive applications.

In a preferred embodiment of the method of the invention, (a) the composition comprising heparin and/or a functional derivative thereof further comprises phosphorylethanolamine and/or a derivative thereof; (b) the composition comprising stabilising excipients further comprises phosphorylethanolamine and/or a derivative thereof; (c) the method further comprises a step (i-1) of overlaying the affinity-reagent-coupled carrier obtained in step (i) of the method of the invention by incubation with a composition comprising phosphorylethanolamine and/or a derivative thereof prior to steps (ii) and (iii); (d) the method further comprises a step of overlaying the heparin-coated affinity-reagent coupled carrier obtained in step (ii) of the method of the invention by incubation with a composition comprising phosphorylethanolamine and/or a derivative thereof; and/or (e) the method further comprises a step of overlaying the affinity-reagent coupled carrier overlaid with stabilising excipients obtained in step (iii) of the method of the invention by incubation with a composition comprising phosphorylethanolamine and/or a derivative thereof. This generates a further overlay which fully or partially covers the preceding overlay(s) and parts of the affinity-reagent and the carrier which are not covered by the previous overlay(s).

Phosphorylethanolamine (PEA) is also known as 2-Aminoethyl dihydrogen phosphate, O-phosphorylethanolamine, O-phosphoethanolamine, phosphoethanolamine or O-phosphocolamine. Its molecular formula is C₂H₈NO₄P and it has a molecular weight of approximately 141 g/mol. Derivatives of PEA include, for example, phosphorylserine, phosphorylcholine, phosphorylinositol, phosphatidylethanolamine, phosphatidylserine, phosphatidylcholine, phosphatidylinositol, lecithin and inositolphosphate. PEA and derivatives thereof can be obtained from commercial suppliers, such as Sigma Aldrich. It is preferred that the composition comprising phosphorylethanolamine and/or derivatives comprises PEA and/or a derivative thereof at a concentration of 5nM-50mM, preferably 50µM-50 mM, more preferably 500µM-5mM. Further, it is preferred that the composition comprising phosphorylethanolamine and/or derivatives thereof is an aqueous solution.

In option (a) PEA and/or a derivative thereof is included in the composition comprising heparin and/or a functional derivative thereof. Similarly, in option (b), PEA and/or a derivative thereof is contained in the composition comprising stabilising excipients.

In accordance with option (c) of this preferred embodiment the affinity-reagent-coupled carrier is overlaid in a step (i-1) by incubation with a composition comprising phosphorylethanolamine and/or derivatives thereof prior to being overlaid by incubation with a composition comprising heparin and/or a functional derivative thereof and by incubation with a composition comprising stabilising excipients. The preceding step of overlaying the the affinity-reagent-coupled carrier by incubation with a composition comprising phosphorylethanolamine and/or derivatives does not affect the order in which the subsequent steps (ii) and (iii) may be performed. In other words, after step (i-1), steps (ii) and (iii) may be performed in the described order ((ii) before (iii)), in reverse order ((iii) before (ii)) or simultaneously.

In accordance with option (d) the step of overlaying the affinity-reagent-coupled carrier with a composition comprising heparin and/or a functional derivative thereof (ii) is followed by a further step of overlaying the obtained carrier by incubation with a composition comprising phosphorylethanolamine and/or derivatives thereof. In other words, a further layer comprising PEA and/or a derivative thereof is applied on top of the layer comprising heparin and/or a functional derivative thereof.

Similarly, in accordance with option (e) the step of overlaying the affinity-reagent-coupled carrier by incubation with a composition comprising stabilising excipients (iii) is followed by a further step of overlaying the obtained carrier by incubation with a composition comprising phosphorylethanolamine and/or derivatives thereof. The order in which steps (ii) and (iii) are carried out does not affect this/these further step(s) of applying (an) additional overlay(s) onto the carrier.

The incubation with a composition comprising phosphorylethanolamine and/or derivatives thereof, alone or in combination with heparin and/or a functional derivative thereof, may be followed by an optional washing and/or drying step. Preferably, only a drying step without a washing step is performed after incubation with a composition comprising PEA and/or a derivative thereof. It is further preferred that the drying step is effected by air-drying.

In accordance with the present invention the preferred method may foremost comprise
1)
   (1) a step (ii) of overlaying the affinity-reagent-coupled carrier obtained in (i) by incubation with a composition comprising heparin and/or a functional derivative thereof and PEA and/or a derivative thereof, and
   (2) a step (iii) of overlaying the heparin- and PEA-coated affinity-reagent-coupled carrier obtained in (ii) by incubation with a composition comprising stabilising excipients; or
2)
   (1) a step (i-1) of overlaying the affinity-reagent-coupled carrier obtained in (i) by incubation with a composition comprising PEA and/or a functional derivative thereof,
   (2) a step (ii) of overlaying the affinity-reagent-coupled carrier overlaid with PEA and/or a derivative thereof obtained in step (i-1) by incubation with a composition comprising heparin and/or a functional derivative thereof, and
   (3) a step (iii) of overlaying the affinity-reagent-coupled carrier overlaid with PEA and/or a derivative thereof and with heparin and/or a functional derivative thereof obtained in step (ii) by incubation with a composition comprising stabilising excipients; or
3)
   (1) a step (ii) of overlaying the affinity-reagent-coupled carrier obtained in (i) by incubation with a composition comprising heparin and/or a functional derivative thereof,
   (2) a step of overlaying the affinity-reagent-coupled carrier coated with heparin and/or a functional derivative thereof obtained in (ii) by incubation with a composition comprising PEA and/or a derivative thereof, and
   (3) a step (iii) of overlaying the heparin-coated affinity-reagent-coupled carrier overlaid with PEA and/or a derivative thereof obtained in the preceding step by incubation with a composition comprising stabilising excipients
4) a step of overlaying the affinity-reagent-coupled carrier obtained in (i) by incubation with a composition comprising heparin and/or a functional derivative thereof, stabilising excipients and PEA and/or a derivative thereof; or
5)
   (1) a step (ii) of overlaying the affinity-reagent-coupled carrier obtained in (i) by incubation with a composition comprising heparin and/or a functional derivative thereof, and
   (2) a step (iii) of overlaying the heparin-coated affinity-reagent-coupled carrier obtained in (ii) by incubation with a composition comprising stabilising excipients, and
   (3) a step of overlaying the affinity-reagent-coupled carrier overlaid with heparin and/or a functional derivative thereof and stabilising excipients by incubation with a composition comprising PEA and/or a derivative thereof; or
6)
   (1) a step (ii) of overlaying the affinity-reagent-coupled carrier obtained in (i) by incubation with a composition comprising heparin and/or a functional derivative, and
   (2) a step of overlaying the affinity-reagent-coupled carrier coated with heparin and/or a functional derivative thereof obtained in (ii) by incubation with a composition comprising stabilising excipients and PEA and/or a derivative thereof; or
7)
   (1) a step (iii) of overlaying the affinity-reagent-coupled carrier obtained in (i) by incubation with a composition comprising stabilising excipients,
   (2) a step (ii) of overlaying the affinity-reagent-coupled carrier overlaid with stabilising excipients obtained in (iii) by incubation with a composition comprising heparin and/or a functional derivative thereof, and
   (3) a step of overlaying the affinity-reagent-coupled carrier overlaid with stabilising excipients and heparin and/or a functional derivative thereof obtained in step (ii) by incubation with a composition comprising PEA and/or a derivative thereof; or
8)
   (1) a step (iii) of overlaying the affinity-reagent-coupled carrier obtained in (i) by incubation with a composition comprising stabilising excipients,
   (2) a step of overlaying the affinity-reagent-coupled carrier overlaid with stabilising excipients and PEA and/or a derivative thereof obtained in (iii) by incubation with a composition comprising PEA and/or a derivative thereof and
   (3) a step (ii) of overlaying the affinity-reagent-coupled carrier overlaid with stabilising excipients and PEA and/or a derivative thereof obtained in the preceding step by incubation with a composition comprising heparin and/or a functional derivative thereof; or
9)
   (1) a step (iii) of overlaying the affinity-reagent-coupled carrier obtained in (i) by incubation with a composition comprising stabilising excipients,
   (2) a step (ii) of overlaying the affinity-reagent-coupled carrier overlaid with stabilising excipients obtained in (iii) by incubation with a composition comprising heparin and/or a functional derivative thereof and PEA and/or a derivative thereof; or
10)
   (1) a step (i-1) of overlaying the affinity-reagent-coupled carrier obtained in (i) by incubation with a composition comprising PEA and/or a functional derivative thereof,
   (2) a step (iii) of overlaying the affinity-reagent-coupled carrier overlaid with PEA and/or a derivative thereof obtained in step (i-1) by incubation with a composition comprising stabilising excipients, and
   (3) a step (ii) a step (ii) of overlaying the affinity-reagent-coupled carrier overlaid with PEA and/or a derivative thereof and with stabilising excipients obtained in step (iii) by incubation with a composition comprising heparin and/or a functional derivative thereof; or
11)
   (1) a step (i-1) of overlaying the affinity-reagent-coupled carrier obtained in (i) by incubation with a composition comprising PEA and/or a functional derivative thereof, and
   (2) a step of of overlaying the affinity-reagent-coupled carrier overlaid with PEA and/or a derivative thereof obtained in step (i-1) by incubation with a composition comprising heparin and/or a functional derivative thereof and stabilising excipients; or
12)
   (1) a step of overlaying the affinity-reagent-coupled carrier obtained in (i) with a composition comprising heparin and/or a functional derivative thereof and stabilising excipients, and
   (2) a step of overlaying the affinity-reagent-coupled carrier overlaid with stabilising excipients and heparin and/or a functional derivative thereof obtained in the preceding step by incubation with a composition comprising PEA and/or a derivative thereof; or
13)
   (1) a step (iii) of overlaying the affinity-reagent-coupled carrier obtained in (i) with a composition comprising stabilising excipients and PEA and/or a derivative thereof, and
   (2) a step of overlaying the affinity-reagent-coupled carrier overlaid with stabilising excipients and PEA and/or a derivative thereof obtained in (iii) by incubation with a composition comprising heparin and/or a functional derivative thereof.

Methods comprising the steps of options 1) to 3) are preferred. Most preferably, the method comprises the steps of option 1).

The carrier produced in accordance with this preferred embodiment of the method of the invention provides reduced unspecific binding as defined herein above. It is preferred that the carrier also provides reduced unspecific binding compared to a reference carrier comprising an overlay comprising heparin and/or a derivative thereof and an overlay comprising stabilising excipients but not comprising phosphorylethanolamine and/or derivatives thereof. Unspecific binding to such a carrier can be assessed by methods well known in the art, such as e.g. essentially the methods described herein above.

It was surprisingly found that the treatment of an affinity-reagent-coupled carrier with a combination of heparin, stabilising excipients and PEA resulted in both reduced unspecific binding and increased specific binding of target molecules. As discussed, such a reduction in unspecific binding and an increase in specific binding are associated with advantages in many scientific as well as therapeutic and diagnostic applications.

The invention further relates a solid carrier comprising (i) at least one affinity-reagent, wherein the at least one affinity-reagent is coupled to the solid carrier, (ii) an overlay comprising heparin and/or a functional derivative thereof, and (iii) an overlay comprising stabilising excipients.

The definitions of terms as provided for the method of the invention as well as any preferred embodiments described in that context apply *mutatis mutandis* also to this carrier unless indicated otherwise.

In particular, as discussed, the overlays need not necessarily be separate. The compounds present in the overlays may instead be comprised in a single overlay. Furthermore, the order of the overlays may be reversed. However, it is preferred that the heparin and/or a functional derivative thereof and the stabilising excipients are comprised in separate overlays and that the overlay comprising stabilising excipients covers the overlay comprising heparin and/or a functional derivative thereof.

It is preferred that the affinity-reagent-coupled carrier is sterile. More preferably the carrier of the invention is a terminally sterilised coated affinity-reagent-coupled carrier. The term "terminally sterilised" in the context of the present invention indicates that the sterilisation is performed after the overlaying steps, i.e. after the affinity-reagent(s) has/have been coupled to the carrier and after the overlay(s) have been applied. Suitable sterilisation methods comprise, but are not limited to ethyleneoxide (EO) treatment, (dry) heat or acid pH treatment, solvent-detergent (SD) treatment, X-rays, autoclaving or plasma sterilisation. Especially preferred is EO or irradiation, mostly preferred beta or gamma irradiation. Also encompassed by the term "terminally sterilised carrier" are carriers which were subjected to a washing step, which was performed under sterile conditions, after sterilisation.

As discussed above with regard to the method of producing the affinity-reagent-coupled carrier of the invention, it was surprisingly found that the combination of an overlay comprising heparin and/or a functional derivative thereof and an overlay comprising stabilising excipients results in the carrier exhibiting a particularly low unspecific binding of non-target molecules and non-target cells and renders the affinity-reagent(s) attached to the carrier less prone to loss of binding activity or affinity of the affinity-reagent(s) during preparation and storage of the carrier. Accordingly, the carrier is sterilisable and can be employed in isolating target molecules or target cells from a sample in high purity. As described above this is associated with many advantages for the scientific understanding of biological processes, as well as for therapeutic, diagnostic, prognostic and potentially predictive applications.

In a preferred embodiment of the carrier of the present invention (a) the overlay comprising heparin and/or a functional derivative thereof and/or the overlay comprising stabilising excipients further comprises phosphorylethanolamine and/or a derivative thereof; or (b) the carrier comprises a further overlay comprising phosphorylethanolamine and/or a derivative thereof which covers the affinity-reagent-coupled carrier and/or one or more of the preceding overlays, if present.

It is preferred that the stabilising excipients and phosphorylethanolamine and/or a derivative thereof are applied in combination, i.e. that the overlay comprising stabilising excipients further comprises phosphorylethanolamine and/or a derivative thereof. In other words, option (a) is particularly preferred.

In accordance with this preferred embodiment the affinity-reagent-coupled carrier may particularly comprise:
1)
   (i) a first overlay comprising heparin and/or a functional derivative thereof and PEA and/or a derivative thereof, and
   (ii) a second overlay comprising stabilising excipients, or
2)
   (i) a first overlay comprising PEA and/or a derivative thereof,,
   (ii) a second overlay comprising heparin and/or a functional derivative thereof, and
   (iii) a third overlay stabilising excipients; or
3)
   (i) a first overlay comprising heparin and/or a functional derivative thereof,
   (ii) a second overlay comprising PEA and/or a derivative thereof, and
   (iii) a third overlay comprising stabilising excipients; or
4) an overlay comprising heparin and/or a functional derivative thereof, stabilising excipients and PEA and/or a derivative thereof; or
5)
   (i) a first overlay comprising heparin and/or a functional derivative thereof,
   (ii) a second overlay comprising stabilising excipients, and
   (iii) a third overlay comprising PEA and/or a derivative thereof; or
6)
   (i) a first overlay comprising heparin and/or a functional derivative thereof,
   (ii) a second overlay comprising PEA and/or a derivative thereof, and
   (iii) a third overlay comprising stabilising excipients; or
7)
   (i) a first overlay comprising stabilising excipients,
   (ii) a second overlay comprising heparin and/or a functional derivative thereof, and
   (iii) a third overlay comprising PEA and/or a derivative thereof; or
8)
   (i) a first overlay comprising stabilising excipients,
   (ii) a second overlay comprising PEA and/or a derivative thereof, and
   (iii) a third overlay comprising heparin and/or a functional derivative thereof; or
9)
   (i) a first overlay comprising stabilising excipients,
   (ii) a second overlay comprising heparin and/or a functional derivative thereof and PEA and/or a derivative thereof; or
10)
   (i) a first overlay comprising PEA and/or a derivative thereof,
   (ii) a second overlay comprising stabilising excipients, and
   (iii) a third overlay comprising heparin and/or a functional derivative thereof; or
11)
   (i) a first overlay comprising PEA and/or a derivative thereof,
   (ii) a second overlay comprising stabilising excipients and heparin and/or a functional derivative thereof; or
12)
   (i) a first overlay comprising stabilising excipients and heparin and/or a functional derivative thereof, and
   (ii) a second overlay comprising PEA and/or a derivative thereof; or
13)
   a first overlay comprising stabilising excipients and PEA and/or a derivative thereof, and
   (ii) a second overlay comprising heparin and/or a functional derivative thereof.

In any one of the options 1 to 13 each overlay fully or partially, preferably fully, covers the preceding overlay, where present, as well as any parts of the affinity-reagent, the carrier or any further preceding overlay which are not covered by the preceding overlay. The combination of overlays of options 1 to 3 is preferred. Most preferred is a carrier that comprises the combination of overlays of option 1.

In a preferred embodiment of the method or the carrier of the invention the first overlay comprises heparin which is selected from the groups consisting of unfractioned heparin, low molecular weight heparin and/or a heparin salt.

The term "unfractionated heparin (UFH)" as used herein refers to polymers consisting of more than 17 units and having a molar mass between 6000 and 30000 Dalton.

The term "low molecular weight heparins (LMWH)" as used herein refers to heparins having a chain length between 5 and 17. Low molecular weight heparins have a molecular weight between approximately 2000 and 8000 Da, typically of about 5000 Dalton. For example, Certoparin (5400 Da), Dalteparin (6100 Da), Enoxaparin (4500 Da), Parnaparin (5000), Nadroparin (4300 Da), Reviparin (4400 Da) and Tinzaparin (6500 Da) are used therapeutically.

The term "heparin salt" refers to forms of heparin which are deprotonated at the ester and amide sulphate groups and are combined with positively charged counter ions. A heparin salt can, for example, be an ammonium heparin salt, a lithium heparin salt or a sodium heparin salt.

In another preferred embodiment of the method or the carrier of the at least one affinity-reagent is coupled to the solid carrier via a bi- or multifunctional bridging molecule.

In a more preferred embodiment of the method or the carrier of the invention the multifunctional bridging molecule is selected from the group consisting of polyethyleneimine, polyhydroxyethylmethacrylate, and polyglycidoxymethacrylate and a mixture thereof.

In another preferred embodiment of the method or the carrier of the invention the at least one affinity-reagent is selected from a group consisting of a (poly)peptide, a nucleic acid molecule, an organic compound and combinations thereof.

The term "peptide" refers to a linear molecular chain of up to 30 amino acids. On the other hand, the term "polypeptide" as used herein interchangeably with the term "protein" describes linear molecular chains of amino acids, including single chain proteins or their fragments, containing more than 30 amino acids. The term "(poly)peptide" as used herein refers to both peptides and polypeptides. (Poly)peptides may further form oligomers consisting of at least two identical or different molecules. The corresponding higher order structures of such multimers are, correspondingly, termed homo- or heterodimers, homo-or heterotrimers etc. Homodimers, trimers etc. of fusion proteins also fall under the definition of the term "(poly)peptide". Furthermore, peptidomimetics of such (poly)peptides where amino acid(s) and/or peptide bond(s) have been replaced by functional analogues are also encompassed by the term (poly)peptide in accordance with the invention. Such functional analogues include all known amino acids other than the 20 gene-encoded amino acids, such as selenocysteine. The term "(poly)peptide" also refers to naturally modified (poly)peptides where the modification is effected e.g. by glycosylation, acetylation, phosphorylation and similar modifications which are well known in the art.

Preferred polypeptides are, for example, antibodies. Alternative preferred polypeptides include alternative binding proteins in which specific binding properties have been introduced, for example by mutagenesis, into a protein scaffold with suitable biophysical properties. Ideally, small globular proteins that are easy to express and purify, which are soluble and stable, which do not aggregate and which are non-immunogenic, are used as a scaffold. So far more than 50 proteins of this class have been described, among them affibodies (which are based on the Z-domain of staphylococcal protein A) adnectins (based on the tenth domain of human fibronectin), anticalins (derived from lipocalins), DARPins (derived from ankyrin repeat proteins), avimers (based e.g. on multimerised Low Density Lipoprotein Receptor (LDLR)-A), nanofitins (derived from the DNA binding protein Sac7d of *Sulfolobus acidocaldarius),* affilins (structurally derived from gamma-B crystalline or ubiquitin), Kunitz domain peptides (derived from the Kunitz domains of various protease inhibitors) and Fynomers^{®} which are derived from the human Fyn SH3 domain (see e.g. Grabulovski et al. (2007) JBC, 282, p. 3196-3204, WO 2008/022759, Bertschinger et al (2007) Protein Eng Des Sel 20(2):57-68, Gebauer and Skerra (2009) Curr Opinion in Chemical Biology 13:245-255, or Schlatter et al. (2012), MAbs 4:4, 1-12). These polypeptides are well known in the art and will be described in further detail herein below.

In accordance with the present invention, the term "nucleic acid molecule" includes DNA, such as cDNA or genomic DNA, and RNA. The term refers *inter alia,* to mammalian, such as human, as well as to bacterial and viral DNA or RNA. Further included are nucleic acid mimicking molecules known in the art such as synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-modified ribonucleic acid such as 2'-O-methoxyethyl ribonucleic acid, morpholino nucleic acid, hexitol nucleic acid (HNA) and locked nucleic acid (LNA) (Braasch DA, Corey DR (2001) Chem Biol. 8(1):1-7). LNA is an RNA derivative in which the ribose ring is constrained by a methylene linkage between the 2'-oxygen and the 4'-carbon. They may contain additional non-natural or derivative nucleotide bases, as will be readily appreciated. The term "nucleic acid molecule" is used interchangeably with the term "polynucleotide" herein.

A polysaccharide is a linear or branched carbohydrate molecule composed of monosaccharide units which are joined by glycosidic bonds.

An "organic compound" in accordance with the present invention is a compound belonging to the class of chemical molecules having a carbon basis. Organic compounds can be natural or synthetic. For example, an organic compound may be a natural or a synthetic antibiotic, such as e.g. lysostaphin or vancomycin.

In a more preferred embodiment of the method or the carrier of the invention, the at least one affinity-reagent is an antibody, a designed ankyrin repeat protein (DARPin), a nanofitin, an anticalin, an aptamer, an adnectin, an affibody, an affilin, an avimer, a Kunitz domain peptide, an antibiotic or a combination thereof.

The term "antibody" as used in accordance with the present invention comprises, for example, polyclonal or monoclonal antibodies. Furthermore, also derivatives or fragments thereof, which still retain the binding specificity, are comprised in the term "antibody". Antibody fragments or derivatives comprise, inter alia, Fab or Fab' fragments, Fd, F(ab')₂, Fv or scFv fragments, single domain V_{H} or V-like domains, such as VhH or V-NAR-domains, as well as multimeric formats such as minibodies, diabodies, tribodies or triplebodies, tetrabodies or chemically conjugated Fab'-multimers (see, for example, Altshuler EP, Serebryanaya DV, Katrukha AG. 2010, Biochemistry (Mosc)., vol. 75(13), 1584 Holliger P, Hudson PJ. 2005, Nat Biotechnol., vol. 23(9), 1126). The term "antibody" also includes embodiments such as chimeric (human constant domain, non-human variable domain), single chain and humanised (human antibody with the exception of non-human CDRs) antibodies.
Various techniques for the production of antibodies are well known in the art and described, e.g. in Altshuler et al., 2010 (Altshuler EP, Serebryanaya DV, Katrukha AG. 2010, Biochemistry (Mosc)., vol. 75(13), 1584). Thus, polyclonal antibodies can be obtained from the blood of an animal following immunisation with an antigen in mixture with additives and adjuvants and monoclonal antibodies can be produced by any technique which provides antibodies produced by continuous cell line cultures. Examples for such techniques are described, e.g. in Harlow E and Lane D, Cold Spring Harbor Laboratory Press, 1988; Harlow E and Lane D, Using Antibodies: A Laboratory Manual, Cold Spring Harbor Laboratory Press, 1999 and include the hybridoma technique originally described by Köhler and Milstein, 1975, the trioma technique, the human B-cell hybridoma technique (see e.g. Kozbor D, 1983, Immunology Today, vol.4, 7; Li J, et al. 2006, PNAS, vol. 103(10), 3557) and the EBV-hybridoma technique to produce human monoclonal antibodies (Cole et al., 1985, Alan R. Liss, Inc, 77-96). Furthermore, recombinant antibodies may be obtained from monoclonal antibodies or can be prepared *de novo* using various display methods such as phage, ribosomal, mRNA, or cell display. A suitable system for the expression of the recombinant (humanised) antibodies may be selected from, for example, bacteria, yeast, insects, mammalian cell lines or transgenic animals or plants (see, e.g., US patent 6,080,560; Holliger P, Hudson PJ. 2005, Nat Biotechnol., vol. 23(9), 11265). Further, techniques described for the production of single chain antibodies (see, inter alia, US Patent 4,946,778) can be adapted to produce single chain antibodies specific for the target of this invention. Surface plasmon resonance as employed in the BIAcore system can be used to increase the efficiency of phage antibodies. Preferably the antibody is directed against a cluster of differentiation (CD) molecule, against a molecule used as differentiation marker to identify e.g. progenitor stem cells or cells from the myeloid lineage, against a cell-membrane-associated adhesion molecule or against a cell membrane molecule relevant for signalling. Such molecules are well known in the art. (Rahman MM, Madlambayan GJ, Cogle CR, McFadden G. (2010), Cytokine Growth Factor Rev. 21(2-3):169-75) More preferably, the antibody is an anti-EpCAM, an anti-Her2 or an anti-Fas (CD95) antibody. For example, the affinity-reagent(s) may be one of the anti-EpCAM antibodies employed in the examples.

In accordance with the present invention, the term "DARPin" refers to a designed ankyrin repeat domain (166 residues), which provides a rigid interface arising from typically three repeated β-turns. DARPins usually carry three repeats corresponding to an artificial consensus sequence, whereby six positions per repeat are randomised. Consequently, DARPins lack structural flexibility (Gebauer and Skerra, 2009). It is preferred that the DARPin is an EpCAM-specific DARPin.

A "nanofitin" (also known as affitin) is an antibody mimetic protein that is derived from the DNA binding protein Sac7d of *Sulfolobus acidocaldarius.* Nanofitins usually have a molecular weight of around 7kDa and are designed to specifically bind a target molecule by randomising the amino acids on the binding surface (Mouratou B, Béhar G, Paillard-Laurance L, Colinet S, Pecorari F., (2012) Methods Mol Biol. ;805:315-31).

The term "anticalin" as used herein refers to an engineered protein derived from a lipocalin (Beste G, Schmidt FS, Stibora T, Skerra A. (1999) Proc Natl Acad Sci U S A. 96(5):1898-903; Gebauer and Skerra (2009) Curr Opinion in Chemical Biology 13:245-255). Anticalins possess an eight-stranded β-barrel which forms a highly conserved core unit among the lipocalins and naturally forms binding sites for ligands by means of four structurally variable loops at the open end. Anticalins, although not homologous to the IgG superfamily, show features that so far have been considered typical for the binding sites of antibodies: (i) high structural plasticity as a consequence of sequence variation and (ii) elevated conformational flexibility, allowing induced fit to targets with differing shape.

An "aptamer" is a DNA or RNA molecule or a peptide that binds other molecules, such as nucleic acids, proteins, small organic compounds, and even entire organisms. A database of aptamers is maintained in the world wide web at aptamer.icmb.utexas.edu/.
More specifically, aptamers can be classified as DNA or RNA aptamers or peptide aptamers. Whereas the former consist of (usually short) strands of oligonucleotides, the latter consist of a short variable peptide domain, attached at both ends to a protein scaffold.
Nucleic acid aptamers are nucleic acid species that have been engineered through repeated rounds of *in-vitro* selection or equivalently, SELEX (systematic evolution of ligands by exponential enrichment) to bind to various molecular targets such as small molecules, proteins and nucleic acids.
Peptide aptamers consist of a variable peptide loop attached at both ends to a protein scaffold. This double structural constraint greatly increases the binding affinity of the peptide aptamer to levels comparable to an antibody's (nanomolar range). The variable loop length is typically comprised of 10 to 20 amino acids, and the scaffold may be any protein which has good solubility properties. Currently, the bacterial protein Thioredoxin-A is the most widely used scaffold protein, the variable loop being inserted within the reducing active site, which is a -Cys-Gly-Pro-Cys- loop in the wild protein, the two cysteins lateral chains being able to form a disulfide bridge. Peptide aptamer selection can be made using different systems, but the most widely used is currently the yeast two-hybrid system.
Aptamers offer the utility for biotechnological and therapeutic applications as they offer molecular recognition properties that rival those of the commonly used biomolecules, in particular antibodies. In addition to their discriminate recognition, aptamers offer advantages over antibodies as they can be engineered completely in a test tube, are readily produced by chemical synthesis, possess desirable storage properties, and elicit little or no immunogenicity in therapeutic or diagnostic applications. Several modifications, such as 2'-fluorine-substituted pyrimidines, polyethylene glycol (PEG) linkage, fusion to albumin or other half life extending proteins etc. are available with which the half-life of aptamers easily can be increased to the day or even week time scale.

An "adnectin" (also referred to as "monobody") in accordance with the present invention, is based on the 10th extracellular domain of human fibronectin III (10Fn3), which adopts an Ig-like β-sandwich fold of 94 residues with 2 to 3 exposed loops, but lacks the central disulphide bridge (Gebauer and Skerra (2009) Curr Opinion in Chemical Biology 13:245-255). Adnectins with the desired target specificity can be genetically engineered by introducing modifications in specific loops of the protein.

The term "affibody" as used herein refers to a family of antibody mimetics which is derived from the Z-domain of staphylococcal protein A. Structurally, affibody molecules are based on a three-helix bundle domain which can also be incorporated into fusion proteins. In itself, an affibody has a molecular mass of around 6kDa and is stable at high temperatures and under acidic or alkaline conditions. Target specificity is obtained by randomisation of 13 amino acids located in two alpha-helices involved in the binding activity of the parent protein domain (Feldwisch J, Tolmachev V.; (2012) Methods Mol Biol.;899:103-26).

The term "affilin" as used herein refers to antibody mimetics that are developed by using either gamma-B crystalline or ubiquitin as a scaffold and modifying amino-acids on the surface of these proteins by random mutagenesis. Selection of affilins with the desired target specificity is effected, for example, by phage display or ribosome display techniques. Depending on the scaffold, affilins have a molecular weight of approximately 10 or 20kDa. As used herein the term affilin also refers to di- or multimerised forms of affilins (Weidle UH, et al., (2013), Cancer Genomics Proteomics;10(4):155-68).

The term "avimer" as used herein refers to a class of antibody mimetics which consist of two or more peptide sequences of 30 to 35 amino acids each which are derived from A-domains of various membrane receptors and which are connected by linker peptides. Binding of target molecules occurs via the A-domain and domains with the desired binding specificity can, for example, be selected by phage display techniques. Binding specificity of the different A-domains contained in an avimer may, but does not have to, be identical (Weidle UH, et al., (2013), Cancer Genomics Proteomics;10(4):155-68).

A "Kunitz domain peptide" is derived from the Kunitz domain of a Kunitz-type protease inhibitor such as bovine pancreatic trypsin inhibitor (BPTI), amyloid precursor protein (APP) or tissue factor pathway inhibitor (TFPI). Kunitz domains have a molecular weight of approximately 6kDA and domains with the required target specificity can be selected by display techniques such as phage display (Weidle et al., (2013), Cancer Genomics Proteomics;10(4):155-68).

An "antibiotic" as referred to herein is a substance that kills bacteria or slows their growth. Preferred antibiotics in accordance with the invention are antibiotics that interact with components of the bacterial cell wall such as for example lysostaphin or vancomycin.

In accordance with the present invention, the term "Fynomer^{®}" refers to a non-immunoglobulin-derived binding polypeptide derived from the human Fyn SH3 domain. Fyn SH3-derived polypeptides are well-known in the art and have been described e.g. in Grabulovski et al. (2007) JBC, 282, p. 3196-3204, WO 2008/022759, Bertschinger et al (2007) Protein Eng Des Sel 20(2):57-68, Gebauer and Skerra (2009) Curr Opinion in Chemical Biology 13:245-255, or Schlatter et al. (2012), MAbs 4:4, 1-12).

In another preferred embodiment of the method or the carrier of the invention the at least one affinity-reagent binds specifically to a biological target molecule on the surface of a target cell or a virus.

In a more preferred embodiment of the carrier or the method of the invention the biological target molecule is a cell membrane molecule and/or the target cell is a circulating tumour cell, an endothelial cell, a foetal cell, a cell of the immune system and/or a precursor cell thereof.

As used herein the term "cell membrane molecule" refers to a molecule that is attached to, associated with or integrated into the cell membrane. Also referred to by this term are molecules that are attached to, associated with or integrated into the cell wall of a microorganism. A cell membrane molecule may, for example, be a membrane protein, a polysaccharide or a lipid. Whereas peripheral membrane proteins are only temporarily attached to the lipid bilayer, integral membrane proteins are permanently attached to the membrane. The latter can be further divided into proteins that span the membrane, i.e. the transmembrane proteins, and proteins that are associated only with the outer of the membrane layers. A membrane protein can for example be a receptor, such as a cytokine or chemokine receptor, a membrane-bound cytokine, an adhesion molecule, a differentiation marker (cluster of differentiation; CD) or an MHC molecule (e.g. HLA).

The term "circulating tumour cell" or "CTC" refers to a cell which has separated from a primary tumour, has entered the vasculature and is circulating in the bloodstream. CTCs have the ability to exit the blood stream again and serve as a seed for subsequent growth of metastases (Baccelli I et al. (2013), Nat Biotechnol.;31 (6):539-44).

The term "endothelial cell" refers to a cell type which usually forms a thin layer that lines the interior surface of vessels such as blood vessels and lymphatic vessels. A "foetal cell" is a cell derived from a foetus.

The term "cell of the immune system" refers to cells involved in protecting an organism from infections. This includes B-cells, T-cells, monocytes/macrophages, dendritic cells, mast cells, granulocytes and natural killer cells. In accordance with the present invention, the term "cell of the immune system" also encompasses platelets as these cells can direct immune responses. The target cell may also be a subtype of any of these cells or a cell of the immune system at a specific stage of differentiation. Cells of the immune system at different stages of differentiation can usually be distinguished by the expression of certain surface markers. Surface markers specific for a particular type of cell of the immune system and for a particular differentiation stage of that cell type are well known in the art. Accordingly, the target cells may for example be neutrophils, basophils, eosinophils, CD4+ T-cells, CD8+ T-cells, regulatory T cells (Tregs), immature dendritic cells, mature dendritic cells, etc.

As used herein, the term "precursor cell" refers to a not fully differentiated cell having the ability to differentiate into a specific type of cell. A precursor cell of a cell of the immune system therefore has the ability to differentiate into that specific type of immune cell. Examples for such precursor cells include dendritic cells, stem cells and cells from the myeloid lineage.

In a preferred embodiment of the method or the carrier of the invention the stabilising excipients protect the at least one affinity-reagent from thermal, irradiation-mediated and/or oxidation-mediated damage.

The term "protects from thermal, irradiation-mediated and/or oxidation-mediated damage" refers to the capability of the stabilising excipient to reduce undesired effects that high temperatures and exposure to irradiation or oxidizing conditions have on the structure and function of the affinity-reagent(s). It is preferred that the stabilising excipient protects the affinity-reagent(s) from damage occurring during sterilisation. This protection may be exerted by protecting the affinity-reagent(s) from thermal, irradiation and/or oxidation mediated damage as well as from other types of damage arising due to the conditions used for sterilisation. In this regard two aspects are envisioned. First, in the presence of the stabilising excipient the capability of the coated affinity-reagent-coupled carrier to specifically bind target molecules or cells after sterilisation or after exposure to high temperatures, irradiation and/or oxidizing conditions is higher as compared to the same coated affinity-reagent-coupled carrier that was sterilised and/or exposed to the same high temperatures, irradiation or oxidizing conditions in the absence of the stabilising excipient. Second, the stabilising excipient ensures that the capability of the coated affinity-reagent-coupled carrier to specifically bind target molecules before sterilisation and/or exposure to high temperatures, irradiation and/or oxidizing conditions is retained, at least partially, also after sterilisation and/or exposure to high temperatures, irradiation and/or oxidizing conditions. With regard to the first aspect the capability of the coated affinity-reagent-coupled carrier to specifically bind target molecules or cells after sterilisation and/or exposure to high temperatures, irradiation or oxidizing conditions in the presence of the stabilising excipient is at least 5% higher as compared to said capability after sterilisation and/or exposure to high temperatures, irradiation and/or oxidizing conditions in the absence of the stabilising excipient, such as at least 10%, such as at least 20%, such as at least 30%, such as at least 40%, such as at least 50%, such as at least 60%, such as at least 70%, such as at least 80%, such as at least 90% as compared to said capability after sterilisation and/or exposure to high temperatures, irradiation and/or oxidizing conditions in the absence of the stabilising excipient. Preferably, the capability of the coated affinity-reagent-coupled carrier to specifically bind target molecules or cells after sterilisation and/or exposure to high temperatures, irradiation or oxidizing conditions in the presence of the stabilising excipient is at least twice as high, such as at least 3-times higher, such as at least 4-times higher such as at least 5-times higher as compared to said capability after sterilisation and/or exposure to high temperatures, irradiation and/or oxidizing conditions in the absence of the stabilising excipient. With regard to the second aspect, in the presence of the stabilising excipient, the coated affinity-reagent-coupled carrier after sterilisation and/or exposure to high temperatures, irradiation and/or oxidizing conditions retains at least 30%, such as at least 40%, such as at least 50%, such as at least 60%, such at least 70%, such as at least 80%, such as at least 90%, such as at least 95%, such as at least 98%, such as at least 99% and most preferably 100% (i.e. fully retains) of the capability of the coated affinity-reagent-coupled carrier to specifically bind target molecules or cells before sterilisation and/or exposure to high temperatures, irradiation and/or oxidizing conditions. In this regard, it will be appreciated that the capability to specifically bind target molecules or cells does not need to be determined for the exact same carrier before and after exposure to high temperatures, irradiation and/or oxidizing conditions but that an identical carrier may be used for comparison purposes. Further, it will be appreciated that the capability of coated affinity-reagent-coupled carrier to specifically bind target molecules or cells can be expressed for example in terms of the amount of target molecules or the number of target cells that can be bound or in terms of the binding affinity.

In a more preferred embodiment of the carrier or the method of the invention the stabilising excipients contain amino acids, peptides, a saponine and/or chitosan.

The term "amino acid", in accordance with the present invention, relates to organic molecules that have a carboxylic acid group, an amino group and a side-chain (also referred to herein as R group) that varies between different amino acids. Amino acids are the essential building blocks of proteins. In accordance with the present invention, the term "amino acid" refers to free amino acids which are not bound to each other to form oligo- or polymers such as dipeptides, tripeptides, oligopeptides or polypeptides.

In accordance with the present invention, the amino acids can be selected from naturally occurring amino acids as well as artificial amino acids or derivatives of these naturally occurring or artificial amino acids.

Naturally occurring amino acids are e.g. the 20 proteinogenic amino acids glycine, proline, arginine, alanine, asparagine, aspartic acid, glutamic acid, glutamine, cysteine, phenylanine, lysine, leucine, isoleucine, histidine, methionine, serine, valine, tyrosine, threonine and tryptophan. Other naturally occurring amino acids are e. g. carnitine, creatine, creatinine, guanidinoacetic acid, ornithine, hydroxyproline, homocysteine, citrulline, hydroxylysine or beta-alanine. Artificial amino acids are amino acids that have a different side chain length and/or side chain structure and/or have the amine group at a site different from the alpha-C-atom. Derivates of amino acids include, without being limiting, n-acetyl-tryptophan, phosphonoserine, phosphonothreonine, phosphonotyrosine, melanin, argininosuccinic acid and salts thereof and DOPA. In connection with the present invention, all the terms also include the salts of the respective amino acids.

The naturally occurring amino acids, but also other than naturally occurring amino acids such as artificial amino acids, can be classified into characteristic groups (Nelson D.L. & Cox M.M., "Lehninger Biochemie" (2005), pp. 122-127): the groups of (a) amino acids with non polar, aliphatic R groups; (b) amino acids with polar, uncharged R groups; (c) amino acids with positively charged R groups; (d) amino acids with negatively charged R groups; and (e) amino acids with aromatic R groups.

Alternatively, one or more of the amino acids can be selected from the group consisting of natural non-proteinogenic amino acids and synthetic amino acids.

The term "non-proteinogenic amino acids", in accordance with the present invention, relates to amino acids which occur in nature but which are not incorporated into polypeptides and proteins. Non-proteinogenic amino acids can be derived from proteinogenic amino acids, which are L-α-amino acids, by post-translational modifications. Such non-proteinogenic amino acids are, for example, lanthionine, 2-aminoisobutyric acid, dehydroalanine, and the neurotransmitter gamma-aminobutyric acid. Also the D-enantiomers of proteinogenic L-amino acids represent non-proteinogenic amino acids. Further non-limiting examples of non-proteinogenic amino acids include carnitine, creatine, creatinine, guanidinoacetic acid, ornithine, hydroxyproline, homocysteine, citrulline, hydroxylysine or beta-alanine.

The term "synthetic amino acids", as used herein, relates to amino acids not naturally occurring in nature. Non-limiting examples of synthetic amino acids include (2R)-amino-5-phosphonovaleric acid, D-phenyl glycine or (S)- and (R)-tert-leucine.

It is further preferred that the stabilising excipients additionally or alternatively comprise at least one peptide. Preferably, the peptide is a di- or tripeptide. Accordingly, in a preferred embodiment of the present invention, the stabilising excipients additionally or alternatively comprise at least one di- or tripeptide. The term "at least one dipeptide or tripeptide" also relates to at least two di- or tripeptides, such as at least three, at least four, at least five, at least six, at least seven, at least eight or at least nine di- or tripeptides. The term further encompasses exactly one, exactly two, exactly three, exactly four, exactly five, exactly six, exactly seven, exactly eight or exactly nine di- or tripeptides. Where more than one di- or tripeptide is comprised in the solution, a mixture of dipeptides and tripeptides is explicitly envisaged herein. The number of di- and tripeptides can be selected independently of each other, e.g. the solution may comprise two dipeptides and three tripeptides. It will be appreciated that when referring to a certain number of di- and tripeptides herein, said number is intended to limit the amount of different types of di- and tripeptides, but not the number of molecules of one type of dipeptide or tripeptide. Thus, for example the term "four dipeptides or tripeptides", refers to four different types of dipeptides and/or tripeptides, wherein the amount of each individual di- and/or tripeptide is not particularly limited. Preferably, the number of (different) di- or tripeptides does not exceed nine di- or tripeptides.

The term "dipeptide or tripeptide", as used herein, relates to peptides consisting of two or three amino acids, respectively. Exemplary dipeptides are glycylglutamine (Gly-Gln), glycyltyrosine (Gly-Tyr), alanylglutamine (Ala-Gln) and glycylglycine (Gly-Gly). Further non-limiting examples of naturally occurring dipeptides are carnosine (*beta*-alanyl-L-histidine), N-acetyl-carnosine (N-acetyl-(*beta-*alanyl-L-histidine), anserine (*beta*-alanyl-N-methyl histidine), homoanserine (N-(4-aminobutyryl)-L-histidine), kyotorphin (L-tyrosyl-L-arginine), balenine (or ophidine) (*beta*-alanyl-N *tau*-methyl histidine), glorin (N-propionyl-*γ*-L-glutamyl-L-ornithine-*δ*-lac ethyl ester) and barettin (cyclo-[(6-bromo-8-en-tryptophan)-arginine]). Examples of artificial dipeptides include, without being limiting, aspartame (*N*-L-a-aspartyl-L-phenylalanine 1-methyl ester) and pseudoproline. A preferred dipeptide is carnosine, which is also referred to as beta-alanyl-L-histidine or (2S)-2-[(3-Amino-1-oxopropyl)amino]-3-(3H-imidazol-4-yl)propanoic acid, and which is a dipeptide consisting of the amino acids beta-alanine and histidine.

Exemplary tripeptides are glutathione (*γ*-glutamyl-cysteinyl-glycine) and its analogues ophthalmic acid (L-*γ*-glutamyl-L-*α*-aminobutyryl-glycine) as well as norophthalmic acid (*y*-glutamyl-alanyl-glycine). Further non-limiting examples of tripeptides include isoleucine-proline-proline (IPP), glypromate (Gly-Pro-Glu), thyrotropin-releasing hormone (TRH, thyroliberin or protirelin) (L-pyroglutamyl-L-histidinyl-L-prolinamide), melanostatin (prolyl-leucyl-glycinamide), leupeptin (N-acetyl-L-leucyl-L-leucyl-L-argininal) and eisenin (pGlu-Gln-Ala-OH). It is preferred that the at least one di- or tripeptide and more preferred all di- or tripeptides, when used in connection with medical applications, do not exert any pharmacological properties.

Preferably, the total amount of all amino acids, dipeptides and/or tripeptides (that is the sum of all of these components in the solution) to be employed is between 0.1 and 150 mg/ml, preferably between 1 and 100 mg/ml, more preferably between 10 and 50 mg/ml, even more preferably between 20 and 35 mg/ml and most preferably the amount is 25 mg/ml.

Saponines are a class of chemical compounds forming secondary metabolites which are found in natural sources, derived from natural sources or can be chemically synthesised. Saponines are found in particular abundance in various plant species. Saponines are amphipathic glycosides grouped phenomenologically by the soap-like foaming they produce when shaken in aqueous solutions, and structurally by their composition of one or more hydrophilic glycoside moieties combined with a lipophilic triterpene derivative. A preferred saponine is glycyrrhizic acid, which is also known as glycyrrhizin, glycyrrhizinic acid or (3β,18α)-30-hydroxy-11,30-dioxoolean-12-en-3-yl 2-O-β-D-glucopyranuronosyl-β-D-glucopyranosiduronic acid and which is a triterpenoid saponin glycoside. Preferably, glycyrrhizic acid is present in a concentration of at least 0.2 g/L, such as at least 0.5g/L, more preferably at least 1 g/L, such as at least 1.5g/L, such as at least 2g/L.
Other examples of saponines include glucuronic acid, escin, hederacoside and digitonin. Further preferred saponines are derivatives of glycyrrhizic acid. Derivatives of glycyrrhizic acid are well-known in the art and include those produced by transformation of glycyrrhizic acid on carboxyl and hydroxyl groups, by conjugation of amino acid residues into the carbohydrate part or the introduction of 2-acetamido-β-D-glucopyranosylamine into the glycoside chain of glycyrrhizic acid. Other derivatives are amides of glycyrrhizic acid, conjugates of glycyrrhizic acid with two amino acid residues and a free 30-COOH function and conjugates of at least one residue of amino acid alkyl esters in the carbohydrate part of the glycyrrhizic acid molecule. Examples of specific derivatives can be found e. g. in Kondratenko et al. (Russian Journal of Bioorganic Chemistry, Vol 30(2), (2004), pp. 148-153).

Chitosan is a polysaccharide composed of randomly distributed β-(1-4)-linked D-glucosamine (deacetylated unit) and N-acetyl-D-glucosamine (acetylated unit). Chitosan is produced commercially by deacetylation of chitin, which is the structural element in the exoskeleton of crustaceans and the cell walls of fungi. The process causes changes in molecular weight and a degree of deacetylation of the product and degradation of nutritionally valuable proteins. The molecular weight of chitosan is between 3800 and 20.000 Daltons. The degree of deacetylation (%DD) can be determined by NMR spectroscopy, and the %DD in commercial chitosans ranges from 60 to 100%. Chitosan can be obtained from commercial suppliers, such as e.g. Heppe Medical Chitosan GmbH, FMC Biopolymer, Sigma Aldrich and others.

The term chitosan, as used herein, encompasses salts and derivatives of chitosan, such as e.g. the salts chitosan-HCl, chitosan-glutamate, chitosan-aspartate, chitosan-citrate, chitosan-acetate, carboxymethyl-chitosan and chitosan derivatives such as trimethyl chitosan, zwitterionic chitosan, and glycated chitosan.

It is known in the art how to choose a suitable chitosan depending on the form of composition employed. For example, where the composition is an aqueous solution, or where the composition is a solution having a pH of 7, the preferred chitosan to be employed is chitosan-HCl.

Preferred amounts of chitosan, and in particular of chitosan-HCl, to be employed are between 0.01 and 15 mg/ml, preferably between 0.1 and 10 mg/ml, more preferably between 0.5 and 5 mg/ml, even more preferably between 1 and 3 mg/ml and most preferably the amount is 2 mg/ml.

It is particularly preferred that the stabilising excipients consist of at least three different amino acids or of two different amino acids and a saponine. In an alternative preferred embodiment the stabilising excipients consist of a combination of amino acids, trehalose and chitosan.

Trehalose is a natural α-linked disaccharide formed by an α,α-1,1-glucoside bond between two α-glucose units. It is also known as mycose or tremalose. Trehalose is well known in the art and can be obtained commercially, for example from Sigma-Aldrich. If present in the composition, trehalose is preferably comprised at a concentration between 0.1 mg/ml to 300mg/ml, more preferably between 80 mg/ml to 200 mg/ml, even more preferably between 100 mg/ml to 180 mg/ml and most preferably about 160 mg/ml.

The term "at least [...] different amino acids" relates to the exact number of amino acids recited but also to more amino acids than recited. For example, the term "at least three different amino acids" also relates to at least four different amino acids, such as at least five, at least six, at least seven, at least eight, at least nine, at least ten different amino acids or more, such as at least eleven, at least 12, at least 13, at least 14, at least 15, at least 16, at least 17 or at least 18 different amino acids. The term further encompasses exactly three, exactly four, exactly five, exactly six, exactly seven, exactly eight, exactly nine, exactly ten, exactly eleven, exactly 12, exactly 13, exactly 14, exactly 15, exactly 16, exactly 17 or exactly 18 different amino acids. Similar considerations apply to the term "at least two different amino acids". It will be readily understood that when referring to an amino acid herein, more than one molecule of said amino acid are intended. Thus, the recited amount of different amino acids is intended to limit the amount of different types of amino acids, but not the number of molecules of one type of amino acid. For example the term "three different amino acids", refers to three different types of amino acids, wherein the amount of each individual amino acid is not particularly limited. Preferably, the number of different amino acids does not exceed 18 different amino acids.

It is further preferred that the at least two amino acids are selected from different groups of the above defined groups of amino acids (a) to (e). For example, when the stabilising excipients comprise two amino acids, the two amino acids are selected from two different groups. For example, one amino acid may be from the group of amino acids with non-polar, aliphatic R groups (a), the other from the group of amino acids with polar uncharged R groups (b) or one amino acid may be from the group of amino acids with positively charged R groups (c) and the other from the group of amino acids with negatively charged R groups (d). Other combinations such as (a)-(c), (a)-(d), (a)-(e), (b)-(c), (b)-(d), (b)-(e), (c)-(e) or (d)-(e) are also explicitly envisaged herein. Similar considerations apply when the stabilising excipients comprise at least three amino acids. In case the stabilising excipients comprise three amino acids, it is preferred that the three amino acids are selected from the above groups (a) to (e). In other words, in this preferred embodiment, when three amino acids are comprised in the solution, the three amino acids may be selected from at least two different groups and, more preferably, from three different groups such that for example one is from group (a), one is from group (b) and one is from group (c). Further combinations such as e.g. (b)-(c)-(d), (c)-(d)-(e), (e)-(a)-(b), (b)-(d)-(e) and so forth are also explicitly envisaged herein. The same consideration applies when the stabilising excipients comprise four amino acids, in which case the amino acids have to be from at least two different groups selected from (a) to (e), more preferably from at least three different groups and most preferably from four different groups. Inter alia, when the stabilising excipients comprise five amino acids, the amino acids have to be from at least two different groups selected from (a) to (e), more preferably from at least three different groups, more preferably from at least four different groups and most preferably from five different groups. The same considerations apply when the stabilising excipients comprise more than five amino acids, such as e.g. six or seven amino acids, in which case these amino acids are selected from at least two different groups selected from (a) to (e), more preferably from at least three different groups, even more preferably from at least four different groups and most preferably from all five different groups.

More preferably, the stabilising excipients comprise at least one amino acid selected from each group of (a) an amino acid with non polar, aliphatic R groups; (b) an amino acid with polar, uncharged R groups; (c) an amino acid with positively charged R groups; (d) an amino acid with negatively charged R groups; (e) an amino acid with aromatic R groups.

Further, it is understood that it is not necessary that the same number of amino acids of each group is present in the solution used according to the invention. Rather, any combination of amino acids can be chosen in accordance with this embodiment as long as at least one amino acid of each group is present.

Alternatively, it is preferred that the stabilising excipients comprise at least the amino acids: (a) alanine, glutamate, lysine, threonine and tryptophane; (b) aspartate, arginine, phenylalanine, serine and valine; (c) proline, serine, asparagine, aspartate, threonine, phenylalanine; (d) tyrosine, isoleucine, leucine, threonine, valine; (e) arginine, glycine, histidin, alanine, glutamate, lysine, tryptophane, or (f) alanine, arginine, glycine, glutamate, lysine.

With regard to this alternative, it is preferred that the stabilising excipients comprise at least the above recited amino acids of either group (a), (b), (c), (d), (e) or (f). In other words, whereas the excipients for stabilisation of the affinity-reagent(s) may comprise more than the above recited amino acids, it is preferred that at least the recited amino acids are present. More preferably, the excipients for stabilisation of the affinity-reagent(s) comprise exactly the recited amino acids and no other amino acids.

In a preferred embodiment of the method of the invention, the method further comprises a step (iv) of sterilising the carrier produced by steps (i) to (iii).

The definition of the term "sterilising" as well as preferred methods for sterilising have been described herein above. It will be appreciated that any steps performed after sterilisation should be performed under sterile conditions in order to maintain sterility.

If step (iii), step (iiib) and/or further steps of overlaying the carrier with PEA and/or a derivative thereof are performed in the method of the invention, then the step of sterilising is performed after this/these step(s).

Due to the embedding in the overlay, the stability of the affinity-reagent(s) is improved, also under sterilisation conditions. Whereas the inventors do not wish to be bound by any theory, it is believed that the latter improvement is achieved by the provision of the overlay which reduces the surface of the affinity-reagent(s) that is accessible to degenerative processes. According to this understanding the embedded affinity-reagent(s) is/are protected and supported in its/their structure by the surrounding overlay.

In a particularly preferred embodiment, the method of the invention comprises the following steps: (a) an optional step of applying PEI to a solid carrier consisting of polyurethane (PU), preferably of a PU foam, under alkaline conditions; (b) a step of applying a pHEMA/pGMA hydrogel to a solid carrier consisting of polyurethane (PU), preferably of a PU foam, or to the carrier obtained in (a); (c) a step of air drying the carrier obtained in (b); (d) a step of coupling an antibody to the carrier obtained in (c), wherein the antibody preferably is an anti-EpCam antibody; (e) a step of overlaying the antibody-coupled carrier obtained in (d) by incubation with a composition comprising heparin, wherein the composition comprising heparin preferably further comprises PEA and/or mPEG-Thiol; (f) a step of washing the carrier obtained in (e); (g) a step of overlaying the carrier obtained in (f) by incubation with a composition comprising stabilising excipients, wherein the composition comprising stabilising excipients preferably comprises glycyrrhicic acid (GA); (h) a step of air drying the carrier obtained in (g); and (i) a step of sterilising the carrier obtained in (h).
More preferably, the method of the invention consists of the above steps. Also preferred is a carrier obtained by the particularly preferred method of the invention described above.

The invention further relates to the use of the carrier of the invention or of the carrier produced by the method of the invention to specifically bind and enrich a target molecule or a target cell which carries a biological target molecule on its surface from a liquid sample.

The definitions of terms as provided for the method of the invention and the carrier of the invention as well as any preferred embodiments described in that context apply *mutatis mutandis* also to the use of the carrier for specifically binding and enriching a target molecule or a target cell which carries a biological target molecule on its surface from a liquid sample unless indicated otherwise.

In accordance with the invention the term "specifically binding and enriching" refers to inducing the specific interaction of a target molecule or a target cell with the coated affinity-reagent-coupled solid carrier and to thereby obtaining a sample, wherein the percentage of target molecules or target cells in the overall content of the sample is increased as compared to the original sample that was contacted with the coated affinity-reagent-coupled solid carrier. Preferably, the percentage of the target molecule or the target cell in the obtained sample is increased by at least 10%, such as at least 20%, such as at least 30%, such as at least 40%, such as at least 50%, such as at least 60%, such as at least 70%, such as at least 80%, such as at least 90%, such as at least 100%, 200%, 300%, 400% or 500% as compared to the original sample. Alternatively it is preferred that the target molecule or the target cell represents at least 70%, such as at least 80%, such as at least 90%, such as at least 95%, such as at least 98%, such as at least 99% of all molecules or cells present in the sample obtained by using the carrier of the invention or the carrier produced by the method of the invention to enrich a target molecule or a target cell which carries a biological target molecule on its surface from a liquid sample. Most preferably, the target molecule or the target cell represents 100% of all molecules or cells in the obtained sample, i.e. the final sample does not contain any other molecules or cells so that the target molecule or the target cell is obtained in a pure form.

The term "liquid sample" defines in the context of the invention fluids that contain the target molecule or the target cell. It may be artificially produced or a naturally occurring or a mix thereof such as e.g. a naturally occurring liquid which has been altered by at least one processing step. An artificially produced liquid sample may for example be a buffered aqueous solution to which molecules or cells have been added. Such buffered aqueous solutions are well known in the art and include, for example, cell culture media such as DMEM and RPMI, phosphate buffer saline (PBS), Tris-based buffers. 4-(2-hydroxyethyl)-1-piperazineethanesulfonic acid, (HEPES), etc. The molecules which are added to such a buffered aqueous solution may, for example, have been produced synthetically, may be derived from *in-vitro* cultures of prokaryotic or eukaryotic cells or may be derived from a sample obtained from a patient, such as, for example, a human patient. Similarly, the cells that are added to the buffered aqueous solution can be derived from *in-vitro* cultures of cells, preferably mammalian cells or can be derived from sample obtained from a patient, such as, for example, a human patient. The sample which is obtained from a patient and from which the molecules or cells may be derived can be solid, e.g. a biopsy such as a tumour biopsy, or liquid, such as blood. Alternatively, the liquid sample is a naturally occurring fluid obtained from a patient, preferably a human patient. Accordingly, the liquid sample may be a body fluid such as blood, plasma, serum, lymph, urine, saliva, ascites, broncho alveolar lavage (BAL), peritoneal fluid or cerebrospinal fluid. Further, the fluid does not need to be a fluid in its naturally occurring form. The liquid sample may, for example, have been subjected to methods of concentrating or diluting. For example, the liquid sample may have been subjected to centrifugation, apheresis, immune precipitation, filtration or other methods in order to enrich the target molecule or target cell.

The invention also relates to the use of the carrier of the invention or of the carrier produced by the method of the invention to specifically induce a biological response in a target cell.

The definitions of terms as provided for the method of the invention, the carrier of the invention and the use of the carrier to specifically bind and enrich a target molecule or a target cell as well as any preferred embodiment described in that context apply *mutatis mutandis* also to the use of the carrier in specifically inducing a biological response in a target cell unless indicated otherwise.

As used herein the term "specifically induce a biological response" refers to provoking a specific reaction in a specific cell such as e.g. cell signalling, wherein said reaction is within the range of reactions which can occur in said cell under physiological conditions. Examples of such biological responses can, for example, be the production of a specific compound, such as for example a cytokine, or the proliferation, the differentiation or the death (e.g. by apoptosis or necrosis) of said target cell.

With regard to this use it is preferred that the biological target molecule is a molecule on the surface of the cell which, upon being bound by the affinity-reagent(s), can transduce a signal to the inside of the cell and initiate the response, for example, via an intracellular signalling cascade. It is also preferred in this regard that the affinity-reagent(s) can not only bind to the biological target molecule but can also induce it to initiate the signal which in turn initiates the biological response, i.e. that the affinity-reagent(s) is an/are agonist(s) with regard to the biological target molecule. It will be appreciated that in order for particular biological responses to be initiated an interplay between more than one biological target molecules may be required. Further, it will be appreciated that the term "initiating a biological response" can also refer to inhibiting target cell specific activities. In such cases the affinity-reagent(s) may be (an) antagonist(s) with regard to the biological target molecule. As an example for inducing a biological response by an agonistic agent, a death ligand, such as e.g. FasL (CD95L) or TRAIL, could be used to bind to its respective death receptor, e.g. Fas (CD95), TRAIL-R1 or TRAIL-R2 and to induce programmed cell death, preferably in the form of apoptosis, in the target cell expressing the receptor. As an alternative example, agonistic antibodies specific for the death receptor can be used for the same purpose. Further, major histocompatibility complex (MHC) molecules charged with a specific peptide and a corresponding co-stimulatory molecule such as e.g. the CD80 and CD86 protein may be used to regulate cytokine production by and proliferation of T cells specific for the presented peptide.

Further, the invention relates an *ex-vivo* or *in-vitro* method for specifically binding and enriching a target cell out of a liquid sample comprising contacting the liquid sample with the carrier of the invention or the carrier produced by the methods of the invention, wherein the contacting is effected by inducing the liquid sample to flow over the surface of the carrier to which the at least one affinity-reagent is coupled, and wherein the target cell carries a biological target molecule on its surface to which the at least one affinity-reagent specifically binds.

The definitions of terms as provided for the method of the invention, the carrier of the invention and the use of the carrier according to the invention as well as any preferred embodiments described in that context apply *mutatis mutandis* also to the *ex-vivo* or *in-vitro* method of the invention unless indicated otherwise.

The term *"ex-vivo* or *in-vitro* method" refers to a method that is carried out outside a living organism. In an *ex-vivo* method living cells or tissues that are derived directly from a living organism, i.e. that have not been outside the organism for more than approximately 24 hours, are employed. In addition, an ex-*vivo* method is performed under conditions that resemble the conditions in a living organism, i.e. the *in-vivo* conditions, as closely as possible. By contrast, in an *in-vitro* method molecules or cells are employed that have not formed part of a living organism for more than 24 hours, or (in case of molecules) that have never formed part of a living organism because they have been produced synthetically. In addition, an *in-vitro* method may be performed under conditions that do not, or at least not closely, resemble the *in-vivo* conditions.

The term "contacting the liquid sample with the carrier" as used herein refers to bringing the liquid sample and the carrier into such close proximity and under such conditions that an interaction between components, such as for example molecules, of the liquid sample and the affinity-reagent(s) coupled to the carrier can take place. Conditions that can influence the interaction between molecules and the affinity-reagent(s) are well known in the art. For example, this includes pH and salt concentration of the liquid, incubation temperature and incubation time as well as the flow rate with which the liquid sample is moved over the carrier.

In accordance with the present invention the term "inducing the liquid sample to flow over the surface of the carrier" relates to ensuring that the liquid sample is in motion relative to the carrier while being contacted with the surface of the carrier to which the affinity-reagent(s) is/are coupled. Preferably, the liquid sample is in a directional motion. In other words, it is preferred that the liquid sample maintains a more or less steady flow into one direction while being contacted with the carrier. Methods for ensuring that a liquid sample is in motion are well known in the art. For example, motion of the liquid sample can be induced by gravity or by the use of a pump. Alternatively, the carrier may be in motion, for example, the carrier may rotate. In either case, measures for ensuring a suitable flow rate are known in the art.

As used with regard to the sample flowing over the carrier, the term "over" does not indicate a spatial restriction. Accordingly, it is not required that the liquid sample is located spatially higher than the carrier. Instead the term "over" indicates that the liquid sample is brought into contact with the carrier in a manner that ensures that the liquid passes along the surface(s) of the carrier to which the affinity-reagent(s) is/are coupled. This can be, for example, achieved by letting the sample flow through a column comprising the carrier or by using a flow chamber (see, e.g., Example 6) or the rotating tube approach as shown in Example 4.

The *ex-vivo* or *in-vitro* method of the present invention may for example be effected by employing a coated affinity-reagent-coupled carrier produced by the method of the present invention as the active part of an apheresis column. The term "active part" as used herein refers to the portion of the apheresis column that is responsible for binding and retaining the target molecule or target cell.

Optionally, the *ex-vivo* or *in-vitro* method further comprises a step of eluting the target molecule or the target cell from the coated affinity-reagent-coupled carrier. The term "eluting" refers to separating the target molecule or the target cell which were bound by the affinity-reagent(s) from the coated affinity-reagent-coupled carrier. This separation is usually achieved by incubating the affinity-reagent-coupled carrier (with the bound target molecules or cells attached thereto) under conditions that are unfavourable for the binding between the affinity-reagent(s) and its/their target(s). Means and methods for achieving a separation of an affinity-reagent from its target are well known in the art and include, for example, altering the pH-value or the salt concentration or adding a substance that binds to the affinity-reagent(s) with similar affinity and is supplied in a much higher concentration (the latter approach being known in the art as competitive elution). Alternatively, the affinity-reagent(s) may comprise a cleavage site. Such a cleavage site can for example be a protease cleavage site and may be naturally present in the affinity-reagent(s) or may have been introduced into the affinity-reagent(s) artificially. In these cases, the target molecule or the target cell may be released from the carrier by adding the cleaving reagent, e.g., a protease. Once the binding has been resolved the target molecule or target cell is physically separated from the affinity-reagent-coupled carrier.

Optionally, the cells bound and isolated by the *ex-vivo* or *in-vitro* method of the present invention may further be expanded after elution from the coated affinity-reagent-coupled carrier. This expansion may on the one hand increase the number of target cells available for further characterisation as described below and may on the other hand represent a step of characterising the target cells in itself. For example, survival and proliferation rates of the target cells may be determined by expanding the target cells.

In a preferred embodiment, the *ex-vivo* or *in-vitro* method of the invention further comprises characterising the target cell by phenotyping and/or genotyping.

The term "characterising the target cell" refers to obtaining information regarding the features of the target cell such as, for example, how it reacts to a specific stimulus, which proteins it expresses and whether its DNA contains any mutations and/or other alterations. This information can be obtained by phenotyping (i.e. examining e.g. the morphology of the cell as well as its protein expression pattern) and/or genotyping (i.e. determining and examining the genetic information) of the cell. Further, the cells may, for example, be investigated for their proliferative, migratory and invasive properties as well as their immunogenicity, tumourigenicity and their ability to metastasise. In addition, the cells may be analysed for their sensitivity for different toxic agents, i.e. it may be determined which agents may be employed to kill the cells, preferably without harming other cell types. This information may, for example, be used in developing new treatments or to determine individualised treatments. Methods to analyse the different properties are well known in the art and include for example, *in-vitro* proliferation, cell death and migration assays (for example through endothelial layers) (Scholz et al., 2000), as well as analysis of the formation of tumours and/or metastases in suitable animal models.

Alternatively or in addition to being analysed by genotyping and/or phenotyping the cells isolated by the *ex-vivo* or *in-vitro* method of the invention may also be employed for therapeutic purposes. For example, the cells may be employed in an adoptive immune transfer or as a vaccine. In the latter case it will be appreciated that, if the isolated cells are potentially harmful, in particular cancerogeneous, they are killed prior to administration and employed as attenuated vaccines.

In addition the invention relates to the carrier of the invention or the carrier produced by the method of the invention for use as an implant or as an extracorporeal device.

As used herein the term "implant" refers to a medical device to be placed in the body of a patient in order to replace a missing biological structure, to support a damaged biological structure or to enhance an existing biological structure. An implant will usually remain in the body of the patient either until it is no longer required, for example due to improvements to the biological structure it was to replace, support or enhance, until it needs to be replaced due to damage to the implant or until the death of the patient. Also encompassed are biodegradable implants, which are degraded, for example, enzymatically.

The term "extracorporeal device" refers to a medical device that is to remain outside of the body of the patient but which is to be connected thereto, for example by being connected to the circulation of a body liquid.

The invention further relates to a module comprising (a) the carrier of the invention or the carrier produced by a method of the invention and (b) a housing surrounding the carrier.

The definitions of terms as provided for the method of the invention, the carrier of the invention, the use of the carrier and the *ex-vivo* or *in-vitro* method of the invention as well as any preferred embodiments described in that context apply *mutatis mutandis* also to module of the invention unless indicated otherwise.

As used herein the term "module" refers to a device comprising different parts. The module comprises a carrier of the invention or a carrier produced by a method of the invention and a housing surrounding the carrier. Preferably, the module further comprises or can be attached to parts of a larger system such as, for example, a medical or diagnostic system such as e.g. an apheresis system.

In accordance with the present invention the term "housing" refers to an object enclosing the carrier, allowing substances to come into contact with the carrier only through the inlet of the housing. It is preferred that the housing has an inlet and an outlet. The terms "inlet" and "outlet" refer to openings in the housing, preferably on opposite sides of the housing. The inlet allows the entry, for example, of a fluid into the housing and the outlet allows the exit of same from the housing. The housing can consist of any solid and insoluble material. Preferably the housing consists of a polymeric material. More preferably, the housing consists of polycarbonate. The carrier is placed inside the housing in a manner that leaves the surface(s) of the carrier to which the affinity-reagent(s) is/are attached accessible to the substances such as e.g. fluids passing through the housing. Preferably the carrier is fixed inside the housing. It is also preferred that the housing has a volume between 10 and 100 mL. The housing can take any suitable shape, it may, for example, be cubic, spherical, prismatic or cylindrical. The design preferably allows for a free flow of whole blood or other body fluids, and the module is preferably rheologically optimised by a minimum pressure difference between the inflow and outflow and more preferably a blood-flow of <1m/sec at any point within the module. Moreover, it is preferred that the housing allows fluids such as e.g. blood to flow through at a flow rate of 10 to 100 mL/min, more preferably at a flow rate of 30 to 50 mL/min. In addition, the inlet and outlet preferably comprise a nozzle that allows the connection of the housing to extracorporeal circuits such as, for example, a standard apheresis systems, for example via a Luer-lock.

In accordance with the present invention, in those cases where different alternatives are recited for a substance, any combination of subject-matter covered thereby is considered to be explicitly disclosed. For example, where for a first substance 1 three alternatives A, B and C, are recited, and for a second substance 2 three alternatives D, E and F are recited, and for a third substance 3 three alternatives G, H and I are recited, then it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, in accordance with the present invention, in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed and also the combination of the subject-matter of claims 3, 2 and 1 is clearly and unambiguously disclosed. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The figures show:

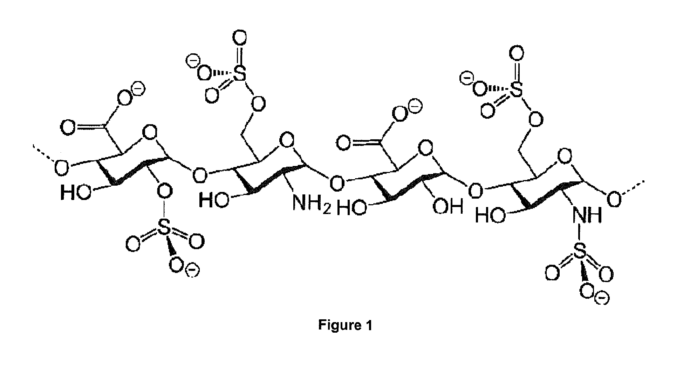

**Figure 1****: Schematic depiction of a part of the chemical structure of heparin.**

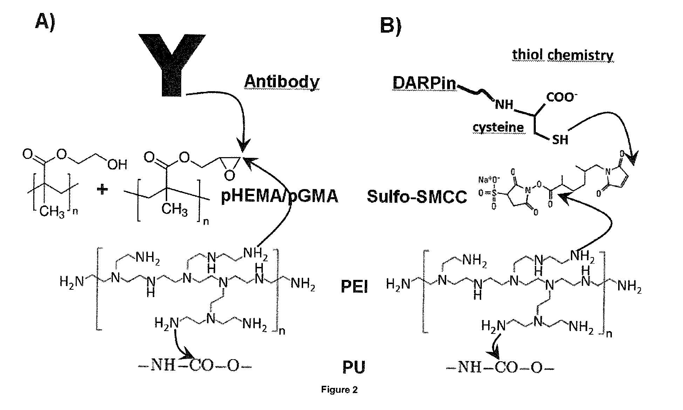

**Figure 2****: Schematic depiction of covalent coupling of affinity-reagents by two different chemical procedures.** A: coupling of an antibody to a polyurethane (PU) carrier via polyethyleneimine (PEI) and a methacrylate hydrogel comprising polyglycidoxy methacrylate (pGMA) and polyhydroxyethyl methacrylate (pHEMA). B: coupling of an EpCAM-specific affinity-reagent (e.g. a designed ankyrin repeated protein (DARPin) molecule) to a PU carrier via PEI and the bifunctional bridging molecule Sulfosuccinimidyl-4-(N-maleimidomethyl)cyclohexane-1-carboxylate (Sulfo-SMCC).

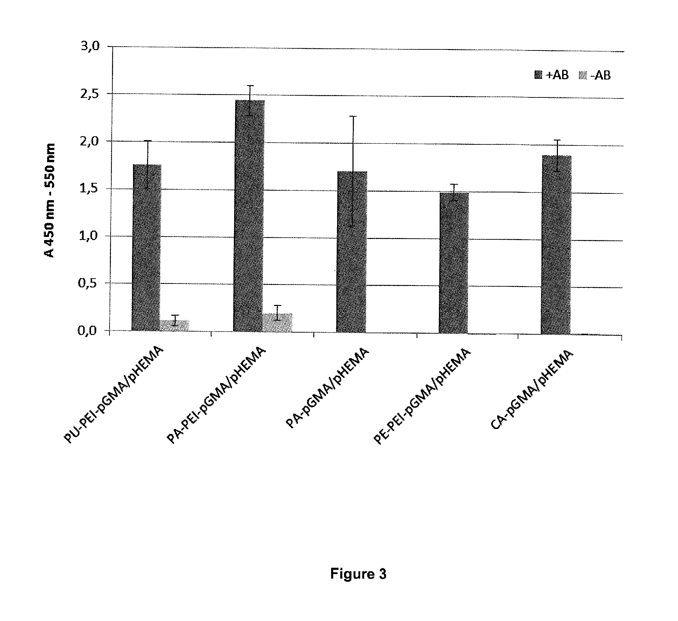

**Figure 3****: Comparison of different coating variants on different supports for the binding of an affinity-reagent.** Materials commonly used in medical devices (polyamide (PA), polyester (PE) and cellulose acetate (CA)) were tested by means of an anti-IgG-HRP assay for the successful coupling of anti-EpCAM-antibodies by two different coating procedures (PEI-pGMA/pHEMA or pGMA/pHEMA). The absorbance (A) was determined at 450 nm and the reference at 550 nm by photometry. +Ab: anti-EpCAM specific antibody was used as an affinity-reagent; -Ab: no antibody was used for control purposes.

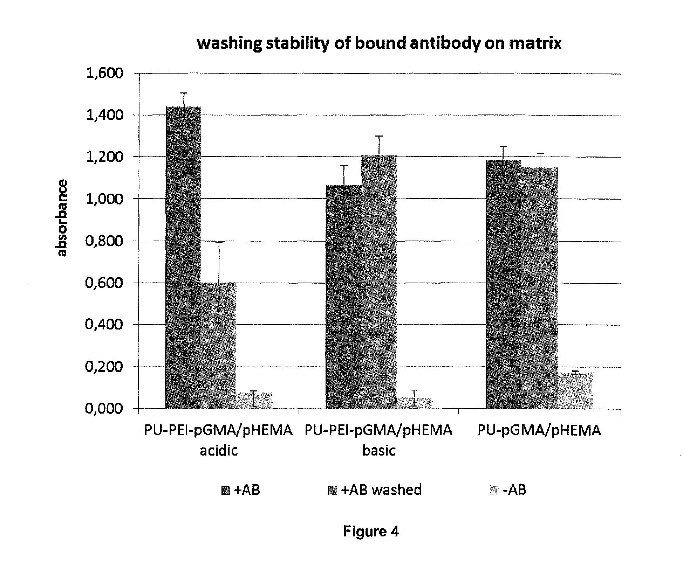

**Figure 4****: Stability of an anti-EpCAM antibody coupled to open porous polyurethane (PU) foams.** Comparison of the effects of different coupling procedures (PEI-pGMA/pHEMA coupling under acidic and basic conditions or pGMA/pHEMA coupling) on coupling stability. Under basic conditions the coupled antibody was stable even after intensive washing overnight. The absorbance (A) was determined at 450 nm and the reference at 550 nm by photometry. +Ab: anti-EpCAM specific antibody was used as an affinity-reagent; -Ab: no antibody was used for control purposes.

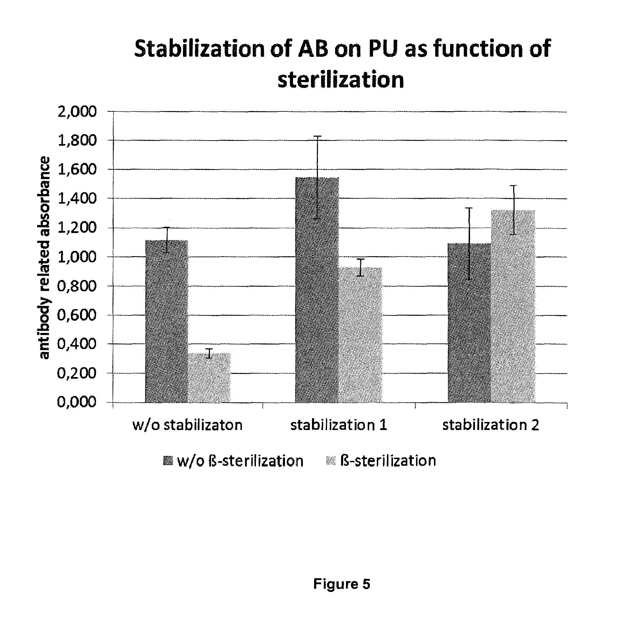

**Figure 5****: Stability of antibodies coupled to a PU carrier under sterilisation conditions (40 kGy β-irradiation).** Two different stabilisation solution variants were tested. Stabilisation 1: without glycyrrhizic acid; Stabilisation 2: with glycyrrhizic acid as stabilising excipient. Stable coupling of anti-EpCAM-antibodies was tested by means of an anti-IgG-HRP assay. The absorbance was determined at 450 nm and the reference at 550 nm by photometry.

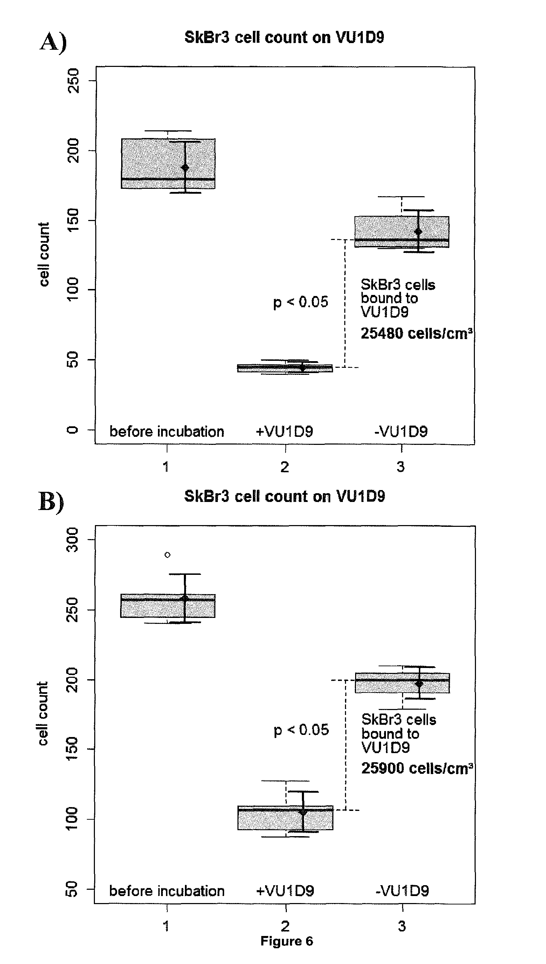

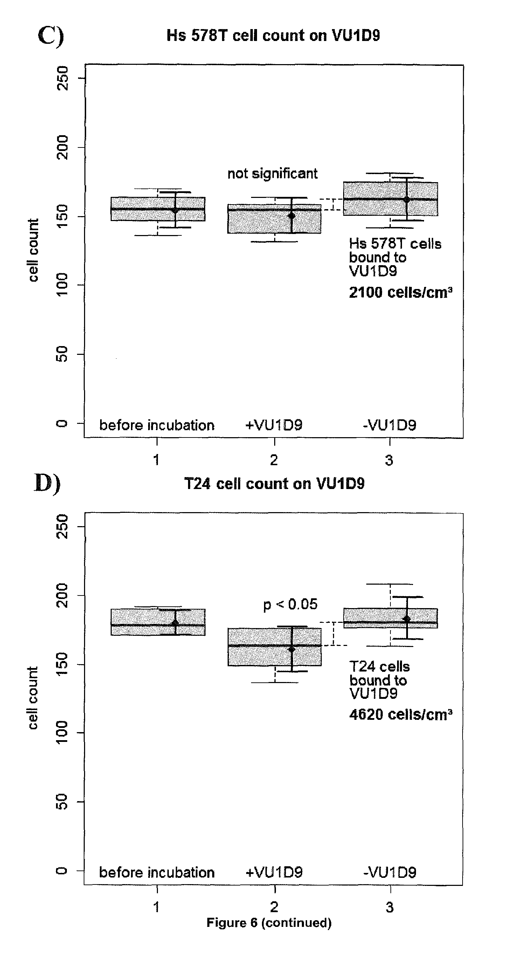

**Figure 6****: Cell counting experiments with "rotating tube assay".** The amount of cells in solutions before incubation, after 60 min of incubation of heparin-blocked matrix in rotating tube, coated with EpCAM specific antibody Vu1D9 and coated w/o antibody (AB), are shown. The difference between the AB- and the AB+ result indicates the cells bound specifically via the AB to the PU. The diamonds indicate the mean values +/- SD being only slightly different from the median values. In total, the coefficients of variation are with about 10% in expected range. A) B) EpCAM+ SkBr3 cells; C) EpCAM-Hs 578T cells; D) EpCAM+/- T24 cells.

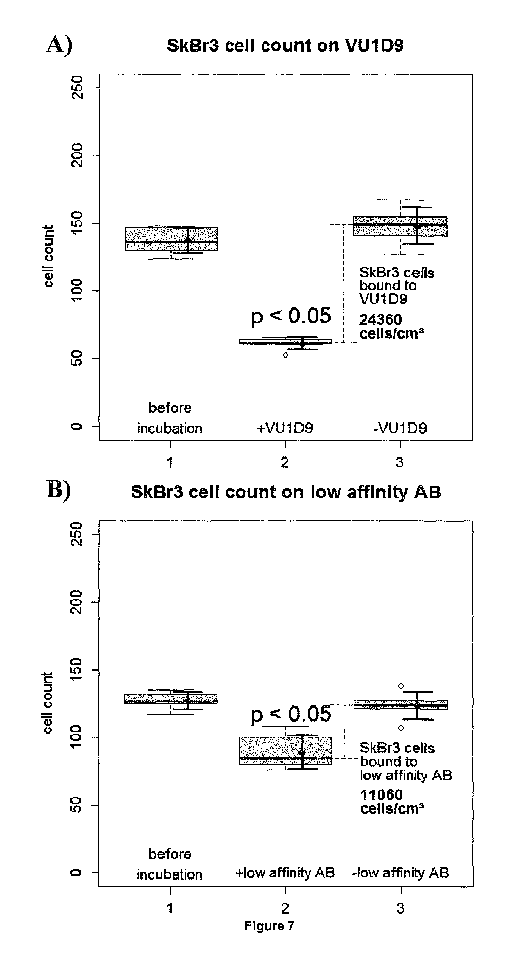

**Figure 7****: Cell counting experiments with "rotating tube assay".** Comparison of the Vu1D9 antibody (A) with a low affinity EpCAM antibody (B).

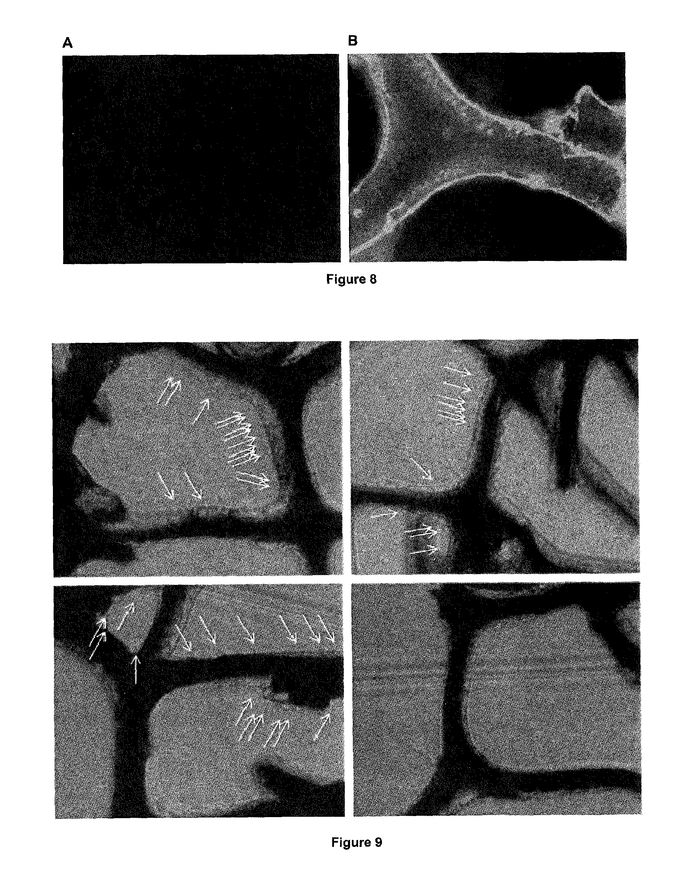

**Figure 8****:** Qualitative detection of homogenous coating on a fragment of PU foam without capture molecule (A) and fluorescently labelled capture molecule (B).
**Figure 9****: Blocking of a matrix with human serum albumin (top) vs. blocking of a matrix with heparin (bottom).** Experiments were done under flowing conditions with slide chambers for microscopy (iBiDi, Munich, Germany). On the left side, the matrix with the covalently bound affinity-reagent and on the right side the matrix without affinity-reagent are shown. In contrast to the HSA blocking which results in unspecific binding of cells, the heparin blocking does not lead to detectable unspecific binding in this assay. Arrows highlight positions of bound cells.

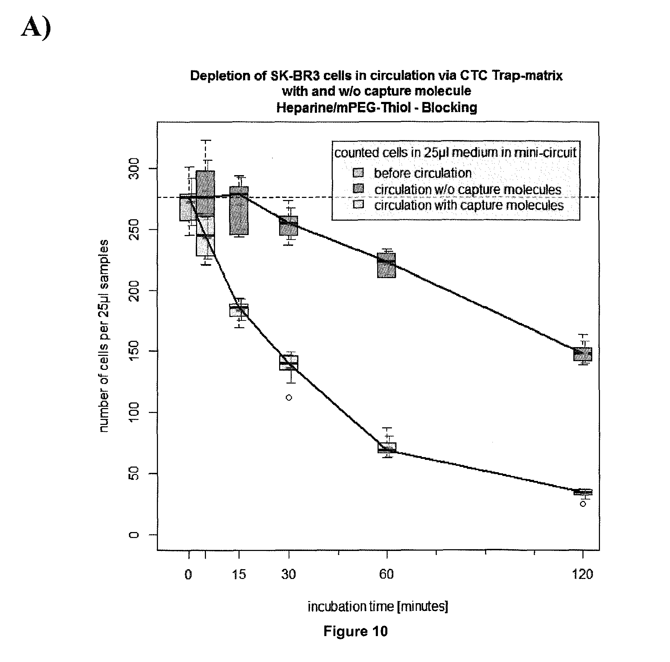

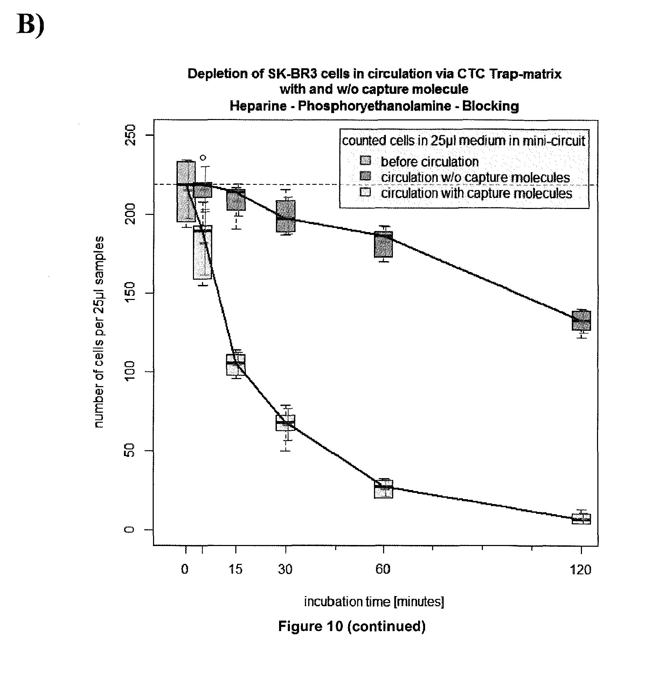

**Figure 10****: Blocking of an affinity-reagent-coupled carrier with heparin and phosphorylethanlolamine reduces unspecific binding of cells even further than blocking with heparin/mPEG-Thiol.** Binding of SK-BR-3 cells in cell culture medium at a concentration of 10000 cells per ml, circulating in bench scale models with and w/o EpCAM specific capture molecules coated to the CTC Trap matrix in the circulation. A total volume of 35 ml is circulated in the setup. The box plots indicate the five value summary of at least 6 measured aliquots of 25 µl each, taken at each time point. The black lines inside the boxes represent the medians, the short gray lines the mean values respectively. Plot A depicts blocking with heparin/mPEG-Thiol, Plot B blocking with heparin and phosphorylethanlolamine. The specific binding of the cells to the capture molecules is higher in the Heparin-PEA approach, especially in the first 30 minutes. In addition, the loss of cells in the circulation without the capture molecules is reduced in case of blocking with a combination of Heparin and phosphorylethanolamine.

The examples illustrate the invention:

### Example 1: Comparison of different antibody coupling matrices on different carrier materials

By means of the anti-IgG-HRP assay the successful coupling of the anti-EpCAM-antibodies using different coupling agents to polyurethane (PU)-foam compared with polyamid (PA)-membranes, polyester (PE)-membranes and cellulose acetate (CA)-membranes was shown. The antibody binding with KOH125/PEI-couplers and subsequent pGMA/pHEMA-coupling (PU, PA and PE) or only pGMA/pHEMA-coupling (PA and CA) was compared. All experiments were done with a positive control (with antibody), a negative control (without antibody), and as triplicates.

PU-foam pieces were cut as cylindrical samples (diameter 1 cm; and 0,5 cm heights). All samples had the same weight (12,0 +/-0,2 mg).

The coupling procedure was carried out in one or two steps. Either foams were treated with or without KOH125/PEI. All samples were treated with pGMA-pHEMA by dip coating and air drying. Blocking of unspecific binding to the carrier was done by incubation of the carrier material with blocking solution overnight and subsequent washing with washing buffer.

For the detection by ELISA, foams were incubated in 1 ml anti-IgG-HRP-solution for at least 1 h on a mini shaker. After washing with washing buffer, foams were incubated with 1 ml TMB (3,3',5,5'-tetramethylbenzidine)-substrate solution in the dark. The intensity of the colour reaction (blue) depended on the antibody (anti-EpCAM) amount and therefore on the density on the surface of the test sample. The addition of the stopping solution (3,6 M sulforic acid) resulted in a colour change (yellow). The absorbance was determined at 450 nm and the reference at 550 nm by photometry. Representative data are shown in Figure 3.

### Example 2: Monitoring of stability of coupling matrix after washing

Covalent attachment of antibody was proven by the method described in Example 1. By means of this method the amount of covalently bound primary antibody per area and volume of foam samples were determined directly after coupling and after a defined number of stringent washing steps. As a negative control, foam with the coupling agent but without antibody was used. To increase the coupling efficacy 1 M ammonium sulphate was added (Salt-induced immobilisation to epoxide, Wheatley and Schmidt, Journal of Chromatography, 644 (1993), p. 11-16). Blocking steps were done by using heparin-ammonium salt. Prolonged washing with PBS for different times was done to confirm the stability of the covalent coupling. The detection was done with a horseradish peroxidase (HRP)-labelled goat antimouse IgG-antibody. The colour reaction of the respective substrate was measured photometrically.

Different coupling protocols with different compositions were compared. The protocol and composition resulting in the highest amount of covalently coupled antibody and minimum loss of antibody during washing were selected for the final covalent coupling method for further use in functional cell capturing assays. In Figure 4, representative data showing the stability of the coupled antibody depending on the treatment protocol (basic conditions prior to acidic conditions) and washing intensity (e.g. overnight) are shown.

### Example 3: Monitoring of stability of coupling matrix after irradiation

The stability of the selected coupling matrix was tested by sterilisation with 40 kGy beta irradiation.

By means of the anti-IgG-HRP assay the stability of the anti-EpCAM matrix was tested. Functionalised foams to which the affinity-reagent was coupled and which were overlaid with heparin were incubated with a stabilising and protecting solution (SPS) with or without 1 g/l glycyrrhizinic acid (GA) for 1 hour, air-dried, and subsequently sterilised with 40 kGy β-irradiation. Two different stabilisation solution variants were tested (stabilisation 1 without and stabilisation 2 with glycyrrhizinic acid). After irradiation, the antibody specific absorbance as measured by indirect photometry was significantly reduced when the matrix was not protected by the stabilising layer. However, stabilisation 1 partially prevented loss of absorbance and stabilisation 2 completely prevented loss of antibody specific absorbance (Figure 5). Table 1 summarises the compatibility of different coupling chemistry strategies as determined in experiments outlined in examples 1-3.

**Table 1: Compatibility of different coupling chemistry compounds with different carrier materials**

| Support | Polymers/chemicals | Support active groups | Protein active groups |
|---|---|---|---|
| PU | PEI/glutaraldehyde | aldehyde | binding to all accessible NH₂ side chains |
| PU | pGMA/pHEMA | epoxide | binding to all accessible NH₂, SH side chains |
| PU | PEI/pGMA/pHEMA | epoxide | binding to all accessible NH₂, SH side chains |
| PU | PDA | catechol | binding to all accessible NH₂, SH side chains |
| PU | PEI/PDA | catechol | binding to all accessible NH₂, SH side chains |
| PU | Glycidoxysilane | epoxide | binding to all accessible NH₂, SH side chains |
| PA | PEI/pGMA/pHEMA | epoxide | binding to all accessible NH₂, SH side chains |
| PA | pGMA/pHEMA | epoxide | binding to all accessible NH₂, SH side chains |
| PE | pGMA/pHEMA | epoxide | binding to all accessible NH₂, SH side chains |
| CA | pGMA/pHEMA | epoxide | binding to all accessible NH₂, SH side chains |
| PU | PEI/Sulfo-SMCC | maleimide | binding to all accessible SH side chains, site specific in case of one Cys |
| PU | PEI/SM(PEG)₁₂ | maleimide | binding to all accessible SH side chains, site specific in case of one Cys |

### Example 4: EpCAM specific cell capturing in a rotating tube assay

Silane-based coupling layers and polymer based coupling layers (PEI, pGMA/ pHEMA, polydopamine - PDA) with different active groups (epoxide, aldehyde) for binding were compared. Additionally, different EpCAM binding molecules (antibody: VU1D9, and EpCAM specific affinity-reagent) were compared for binding to the carrier and for binding of cells with different EpCAM densities. Cells were incubated with the coated foams for 1 h. The total number of trapped cells was approximately 25.000 cells/cm³ PU with the high EpCAM expressing cell line SkBr3 and 4500 cells/cm³ with the low expressing EpCAM cell line T24. The Hs578T cell line (no EpCAM expression) was used as a negative control. Indirect quantification of cells absorbed by the functionalised foams was carried out by counting the cells in the eluate with a Nageotte-counting chamber. As a negative control, coated foams without antibodies were used. In some experiments, the medium expressing EpCAM cell line PC3-9 was used. Representative data from these experiments are shown in Figures 6 and 7.

### Example 5: Detection of homogenous coating on PU foam surface.

Alexa-488-labelled EpCAM-affinity-reagents were used for semi-quantitative detection of coupled affinity-reagents to PU-foams. Affinity-reagents were coupled to PU-foams via SM(PEG)12 linker. The visualisation of the coupled anti-EpCAM affinity-reagents to PU-foams was done by fluorescence microscopy.
One foam was coupled with fluorescence-labelled anti-EpCAM affinity-reagent-Alexa488. Another foam was treated identically, except that no fluorescence-labelled affinity-reagent was used. As an additional control, a foam without any treatment was used. A representative microphotograph is shown in Figure 8.

### Example 6: EpCAM specific cell capturing in a microchamber flow assay.

In this experiment, the binding of cells to the anti-EpCAM molecules coupled to PU-foams were visualised in a flow system (flow chambers from iBiDi). To increase the coupling efficacy 1 M ammonium sulphate was added (Salt-induced immobilisation to epoxide, Wheatley and Schmidt, Journal of Chromatography, 644 (1993), p. 11-16).
Two subsequent blocking steps are done to avoid unspecific binding of cells to the PU-foam, the first with 5 mg/ml Heparin (Heparin-ammonium salt) and the second with a Tris-buffer with SPS. As a comparison a second PU-foam was subjected to a blocking step with human serum albumin (HSA) and a second blocking step with a Tris-buffer with SPS. Heparin is negatively charged and may therefore prevent cell binding to the material surface.

### Example 7: Blocking studies to limit unspecific cell binding

In the experiments outlined in examples 1-6, the compositions and coupling protocols showed the best results when heparin was used as blocking reagent. Therefore, different blocking reagents were systematically compared for testing specific/unspecific binding of cells to the anti-EpCAM tumour cell capture matrix. In Table 2, the comparison of different blocking methods is shown as a summary based on experiments outlined in examples 1-6.

**Table 2. Comparison of different blocking methods to prevent unspecific cell binding**

| *couplers*/*Blocking methods* | *Description of results* |
|---|---|
| *Silane couplers* | *low number of binding cells* |
| | *mostly specific binding* |
| | *low reproducibility* |
| *PEI*/*pGMA*/*pHEMA couplers* | *high number of binding cells* |
| | *low specificity* |
| | *slightly variable by different blocking methods* |
| polydopamine *(PDA) couplers* | *high number of binding cells* |
| | *low specificity* |
| | *variable by different blocking methods* |
| *HSA blocking* | *with pGMA*/*pHEMA and PDA high number of cells binding to both, the matrix with (AB+) and without an antibody (AB-)* |
| *SPS blocking* | *no significant change compared to HSA* |
| *mPEG blocking* | *lower number of cells as with HSA or SPS not fully specific* |
| *Heparin blocking* | *lower number of cells as with HSA or SPS highly specific* |
| *Two step blocking: 1. Heparin blocking with mPEG-Thiol; 2. SPS, PEA, HSA blocking* | *lower number of unspecific cells* as *with heparin alone, HSA or SPS. Increased specific binding to target molecule nearly fully specific* |

### Example 8: Blocking studies to limit unspecific cell binding with cells in circulation

In this experiment, the binding of cells to the anti-EpCAM molecules coupled to PU-foams were measured in a circulating flow system. The amount of cells in the circulating medium was determined by means of cell counting using FACS analysis in aliquots of 25 µl at different time points as depicted in Figure 10. Two subsequent blocking steps are done with 700 IE/ml heparin or 700 IE/ml heparin together with 5 mg/ml mPEG-Thiol and in a second step a Tris-buffer with SPS, and human serum albumin (HSA) with or without 5 mM phosphorylethanolamine to avoid unspecific binding of cells to the PU-foam. Heparin and phosphorylethanolamine are negatively charged and may therefore prevent cell binding to the material surface. In addition, the surprising observation of increase in specific cell binding might be explained by the Heparin-PEA-mediated covering of the binding site resulting in increased competitive binding of cells to the binding site. The compositions of the blocking solutions showed the best results when heparin in combination with phosphorylethanolamine (PEA) was used as blocking reagent, in a way that in a first step the heparin is applied and in a second step the PEA is applied together with HSA and SPS.

### Further References

Amadori, A., E. Rossi, et al. (2009). "Circulating and disseminated tumor cells in the clinical management of breast cancer patients: unanswered questions." Oncology 76(6): 375-386.
Braun, S., F. D. Vogl, et al. (2005). "A pooled analysis of bone marrow micrometastasis in breast cancer." N Engl J Med 353(8): 793-802.
Cohen, S. J., C. J. Punt, et al. (2008). "Relationship of circulating tumor cells to tumor response, progression-free survival, and overall survival in patients with metastatic colorectal cancer." J Clin Oncol 26(19): 3213-3221.
Cristofanilli, M., G. T. Budd, et al. (2004). "Circulating tumor cells, disease progression, and survival in metastatic breast cancer." N Engl J Med 351(8): 781-791.
Danila, D. C., G. Heller, et al. (2007). "Circulating tumor cell number and prognosis in progressive castration-resistant prostate cancer." Clin Cancer Res 13(23): 7053-7058.
de Bono, J. S., G. Attard, et al. (2007). "Potential applications for circulating tumor cells expressing the insulin-like growth factor-I receptor." Clin Cancer Res 13(12): 3611-3616.
de Bono, J. S., H. I. Scher, et al. (2008). "Circulating tumor cells predict survival benefit from treatment in metastatic castration-resistant prostate cancer." Clin Cancer Res 14(19): 6302-6309.
Fernández C, Hattan CM, Kerns RJ (2006) "Semi-synthetic heparin derivatives: chemical modifications of heparin beyond chain length, sulfate substitution pattern and N-sulfo/N-acetyl groups." Carbohydr Res. 341(10):1253-65. Epub 2006 May 18.
Hofman, V., M. I. Ilie, et al. (2011). "Detection of circulating tumor cells as a prognostic factor in patients undergoing radical surgery for non-small-cell lung carcinoma: comparison of the efficacy of the CellSearch Assay and the isolation by sise of epithelial tumor cell method." Int J Cancer 129(7): 1651-1660.
Husemann, Y., J. B. Geigl, et al. (2008). "Systemic spread is an early step in breast cancer." Cancer Cell 13(1): 58-68.
Krebs, M. G., R. Sloane, et al. (2011). "Evaluation and prognostic significance of circulating tumor cells in patients with non-small-cell lung cancer." J Clin Oncol 29(12): 1556-1563.
Meng, S., D. Tripathy, et al. (2004). "HER-2 gene amplification can be acquired as breast cancer progresses." Proc Natl Acad Sci U S A 101(25): 9393-9398.
Morgan, T. M., P. H. Lange, et al. (2009). "Disseminated tumor cells in prostate cancer patients after radical prostatectomy and without evidence of disease predicts biochemical recurrence." Clin Cancer Res 15(2): 677-683.
Muller, V. and K. Pantel (2004). "Bone marrow micrometastases and circulating tumor cells: current aspects and future perspectives." Breast Cancer Res 6(6): 258-261.
Neumann, P. J., J. T. Cohen, et al. (2012). "Willingness-to-pay for predictive tests with no immediate treatment implications: a survey of US residents." Health Econ 21 (3): 238-251.
Pantel, K. and R. H. Brakenhoff (2004). "Dissecting the metastatic cascade." Nat Rev Cancer 4(6): 448-456.
Rossi E., U. Basso, et al. (2010). "M30 neoepitope expression in epithelial cancer: quantification of apoptosis in circulating tumor cells by CellSearch analysis." Clin Cancer Res 16(21): 5233-5243.
Sastre, J., M. L. Maestro, et al. (2008). "Circulating tumor cells in colorectal cancer: correlation with clinical and pathological variables." Ann Oncol 19(5): 935-938.
Scholz M, Blaheta RA, Wittig B, Cinatl J, Vogel JU, Doerr HW, Cinatl J Jr. (2000) Cytomegalovirus-infected neuroblastoma cells exhibit augmented invasiveness mediated by beta1alpha5 integrin (VLA-5). Tissue Antigens. 55(5):412-21.
Wang, L. H., T. D. Pfister, et al. (2010). "Monitoring drug-induced gammaH2AX as a pharmacodynamic biomarker in individual circulating tumor cells." Clin Cancer Res 16(3): 1073-1084.

## Claims

1. A method of producing a solid carrier, to which at least one affinity-reagent is coupled that binds specifically to a target molecule, comprising the steps of
(i) coupling at least one affinity-reagent to a solid carrier,
(ii) overlaying the affinity-reagent-coupled carrier obtained in (i) by incubation with a composition comprising heparin and/or a functional derivative thereof, and
(iii) overlaying the affinity-reagent-coupled carrier by incubation with a composition comprising stabilising excipients,
wherein steps (ii) and (iii) are carried out subsequently or simultaneously and subsequently to step (i).

2. The method of claim 1,
(a) wherein the composition comprising heparin and/or a functional derivative thereof further comprises phosphorylethanolamine and/or a derivative thereof;
(b) wherein the composition comprising stabilising excipients further comprises phosphorylethanolamine and/or a derivative thereof;
(c) further comprising a step (i-1) of overlaying the affinity-reagent-coupled carrier obtained in claim 1 (i) by incubation with a composition comprising phosphorylethanolamine and/or a derivative thereof prior to steps (ii) and (iii);
(d) further comprising a step of overlaying the heparin-coated affinity-reagent coupled carrier obtained in claim 1 (ii) by incubation with a composition comprising phosphorylethanolamine and/or a derivative thereof; and/or
(e) further comprising a step of overlaying the affinity-reagent coupled carrier overlaid with stabilising excipients obtained in claim 1 (iii) by incubation with a composition comprising phosphorylethanolamine and/or a derivative thereof.

3. A solid carrier, comprising
(i) at least one affinity-reagent, wherein the at least one affinity-reagent is coupled to the solid carrier,
(ii) an overlay comprising heparin and/or a functional derivative thereof, and
(ii) an overlay comprising stabilising excipients.

4. The solid carrier of claim 3, wherein
(a) the overlay comprising heparin and/or a functional derivative thereof and/or the overlay comprising stabilising excipients further comprises phosphorylethanolamine and/or a derivative thereof; or
(b) the carrier comprises a further overlay comprising phosphorylethanolamine and/or a derivative thereof which covers the affinity-reagent-coupled carrier and/or one or more of the preceding overlays, if present.

5. The method of claim 1 or 2 or the carrier according to claim 3 or 4, wherein the heparin is selected from the groups consisting of unfractioned heparin, low molecular weight heparin and/or a heparin salt.

6. The method according to claim 1, 2 or 5 or the carrier according to claim 3 to 5, wherein the at least one affinity-reagent is coupled to the solid carrier via a bi- or multifunctional bridging molecule.

7. The method or the carrier according to claim 6, wherein the multifunctional bridging molecule is selected from the group consisting of polyethyleneimine, polyhydroxyethylmethacrylate, and polyglycidoxymethacrylate and a mixture thereof.

8. The method according to any one of claims 1, 2 or 5 to 7 or the carrier according to any one of claims 3 to 7, wherein the at least one affinity-reagent is selected from a group consisting of, a (poly)peptide, a nucleic acid molecule, an organic compound and combinations thereof.

9. The method or the carrier according to claim 8, wherein the at least one affinity-reagent is an antibody, a designed ankyrin repeat protein (DARPin), a nanofitin, an anticalin, an aptamer, an adnectin, an affibody, an affilin, an avimer, a Kunitz domain peptide, an antibiotic or a combination thereof.

10. The method according to any one of claims 1, 2 or 5 to 9 or the carrier according to any one of claims 3 to 9, wherein the at least one affinity-reagent binds specifically to a biological target molecule on the surface of a target cell or a virus.

11. The method or the carrier according to claim 10, wherein the biological target molecule is a cell membrane molecule and/or wherein the target cell is a circulating tumour cell, an endothelial cell, a foetal cell, a cell of the immune system and/or a precursor cell thereof.

12. The method according to any one of claims 1, 2 or 5 to 11 or the carrier according to any one of claims 3 to 11, wherein the stabilising excipients protect the at least one affinity-reagent from thermal, irradiation-mediated and/or oxidation-mediated damage.

13. The method or the carrier according to claim 12, wherein the stabilising excipients contain amino acids, peptides, a saponine and/or chitosan.

14. The method according to any one of claims 1, 2 or 5 to 13, further comprising a step (iv) of sterilising the carrier produced by steps (i) to (iii).

15. Use of the carrier according to any one of claims 3 to 13 or of the carrier produced by the method of any one of claims 1, 2 or 5 to 14 to specifically bind and enrich a target molecule or a target cell which carries a biological target molecule on its surface from a liquid sample.

16. Use of the carrier according to any one of claims 3 to 13 or of the carrier produced by the method of any one of claims 1, 2 or 5 to 14 to specifically induce a biological response in a target cell.

17. An *ex-vivo* or *in-vitro* method for specifically binding and enriching a target cell out of a liquid sample comprising contacting the liquid sample with the carrier of any one of claims 3 to 13 or the carrier produced by the method according to any one of claims 1, 2 or 5 to 14, wherein the contacting is effected by inducing the liquid sample to flow over the surface of the carrier to which the at least one affinity-reagent is coupled, and wherein the target cell carries a biological target molecule on its surface to which the at least one affinity-reagent specifically binds.

18. The carrier according to any one of claims 3 to 13 or the carrier produced by the method of any one of claims 1, 2 or 5 to 14 for use as an implant or as an extracorporeal device.
